(19) Europäisches Patentamt
European Patent Office
Office européen des brevets

(11) EP 4 763 232 A1

(12) **EUROPEAN PATENT APPLICATION**

(43) Date of publication:
**24.06.2026 Bulletin 2026/26**

(21) Application number: **24307284.0**

(22) Date of filing: **23.12.2024**

(51) International Patent Classification (IPC):
*A61K 51/06* (2006.01) *A61K 51/12* (2006.01)
*A61P 35/00* (2006.01) *A61K 103/32* (2006.01)

(52) Cooperative Patent Classification (CPC):
**A61K 51/065; A61K 51/1213; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Centre National de la Recherche Scientifique 75016 Paris (FR)**
- **Université de Lorraine 54000 Nancy (FR)**
- **UNIVERSITE CLAUDE BERNARD - LYON 1 69100 Villeurbanne (FR)**
- **Nano-H 69270 Fontaines-Saint-Martin (FR)**
- **Posifit 54600 Villers-Lès-Nancy (FR)**

(72) Inventors:
- **DAOUK, Joel 54410 LANEUVEVILLE DEVANT NANCY (FR)**
- **RECH, Fabien 54000 NANCY (FR)**
- **PIERSON, Julien 88000 EPINAL (FR)**
- **BARBERI-HEYOB, Muriel 54200 TOUL (FR)**
- **SCHNELLER, Perrine 57430 WILLERWALD (FR)**
- **TILLEMENT, Augustin 69270 FONTAINES SAINT-MARTIN (FR)**
- **LUX, François 69100 VILLEURBANNE (FR)**
- **GRÉA, Thomas 01440 VIRIAT (FR)**
- **TILLEMENT, Olivier 69270 FONTAINES SAINT-MARTIN (FR)**
- **KARCHER, Gilles 54130 SAINT-MAX (FR)**
- **COLLET, Charlotte 54113 BLENOD LES TOUL (FR)**
- **BÉEN, Quentin 54230 CHALIGNY (FR)**

(74) Representative: **Plasseraud IP
104 Rue de Richelieu
CS92104
75080 Paris Cedex 02 (FR)**

(54) **SYSTEM FOR PREPARING A PHARMACEUTICAL RADIOACTIVE HYDROGEL AND PHARMACEUTICAL COMPOSITION FOR TREATMENT OF SOLID CANCER**

(57) The present invention relates to a system for preparing a pharmaceutical radioactive hydrogel comprising (i) at least one hydrophilic polymer, particularly at least one polysaccharide derived from chitosan or hyaluronic acid, bearing at least one chelating group and (ii) at least one therapeutical radionuclide chelatable with said chelating group. It further relates to pharmaceutical compositions comprising said hydrophilic polymer and said therapeutical radionuclide chelated with the chelating group Rc of said hydrophilic polymer, their use for treatment of solid cancer, preferably by brachytherapy.

EP 4 763 232 A1

## Description

## FIELD OF THE INVENTION

The present invention relates to the field of pharmaceutical radioactive hydrogels and pharmaceutical compositions thereof, comprising at least one biocompatible hydrophilic polymer bearing at least one chelating group which can chelate pharmaceutical active principles, especially therapeutical radionuclides, and uses thereof, particularly for the treatment of solid cancer.

In particular, the present invention relates to systems for preparing these pharmaceutical compositions which may form or remain a pharmaceutical hydrogel in physiological conditions, which hydrogel being biodegradable *in vivo.*

The particularly concerned biocompatible hydrophilic polymers are polysaccharides, notably chitosans and hyaluronic acids.

The present invention also pertains to methods for preparing these pharmaceutical compositions by means of these systems.

## BACKGROUND

Cancer remains one of the leading causes of morbidity and mortality worldwide. Various therapeutic options are available depending on the type, localization, stage of cancer.

Radiation therapy is one of the available options, often used in combination with surgery and/or chemotherapy, to reduce the size of the tumor and kill cancer cells. The radiation therapy is most commonly used as external radiation. However, this approach has significant side effects as non-tumor tissues are also damaged by the radiation, leading to radionecrosis, and potentially causing mutations that may result in radiation-induced cancers

Brachytherapy is another option for some kind of cancers. It is a form of internal radiotherapy involving placing a radioactive source directly within or close to the tumor. This allows high-dose radiation delivery while minimizing exposure to surrounding tissues. Brachytherapy is employed in several cancer types, including, without limitation, breast cancers, head and neck cancers, prostate cancers, gynecological cancers and cerebral tumors.

Brachytherapy is notably used in glioblastoma, the commonest primary malignant brain tumor in adults with a very poor prognosis. Different strategies of intracavitary brachytherapy of glioblastoma were suggested such as GliaSite® device, a balloon filled with $^{125}$I to deliver local radiotherapy. However, all available studies suggested that $^{125}$I was associated with an increased risk of radio necrosis and side effects, requiring additional heavy surgery to remove the device.

In recent years, Cesium-131 ($^{131}$Cs) has emerged as a promising radionuclide. Although it shares many characteristics with $^{125}$I, half-life is significantly shorter, improving safety profile. The latest innovation concerns a collagen tile, containing $^{131}$Cs seeds; tiles are positioned in contact with the excision cavity (GammaTile®) (Ringel F et al. Neuro Oncol 2016;18:96-104). This device, approved by FDA, is currently under clinical trial for newly diagnosed glioblastomas. A single tile contains four $^{131}$Cs radioactive seeds at the cavity surface. The delivered dose cannot be adjusted accurately as each seed has an identical radioactive activity. Moreover, it is unclear if the tiles fully conform to the shape of the surgical resection cavity, properly fit the surface, and remain in position, which could lead to an inappropriate dose to infiltrating tumor cells.

The resilience of glioblastoma due to diffuse infiltration has increased investigation into mechanisms of motility and invasion, with the goal of arresting or slowing glioma spread. This therapeutic goal may allow for more successful localized treatment with reduced recurrence.

Brachytherapy strategies based on radioactive chitosan have been described in the prior art. The US patent US 5,762,903 discloses an internal radiation therapeutic composition which is administrable in an aqueous solution and which consists essentially of an active radionuclide directly bonded to chitosan ($^{166}$Ho-Chitosan Complex), said composition formed such that upon contact with higher pH of physiological conditions, said aqueous solution is converted to a stiff gel form in which the active radionuclide does not migrate into surrounding healthy tissue, wherein the radionuclide is selected from the lanthanide series. This chitosan exists in solution in acidic pH and changes into gel and into particles (macroaggregate) in higher pH. $^{166}$Ho-Chitosan Complex is prepared by adding a $^{166}$Ho(NO3)3.5H2O solution to a chitosan (molecular weight in about 500,000; Hydrolysis is about 85%) solution. Chitosan of which the free amines forms chelate with radionuclide is presented as an internal radiation therapeutic agent which treats the lesion in the body and then excretes out of the body. The radioactive chitosan complex and its macroaggregate can be used as an internal radiation therapy for rheumatoid arthritis, liver cancer, brain cancer, breast cancer, ovary cancer and the like by administering directly to the lesion. Nevertheless, the U $^{166}$Ho-Chitosan Complex in this patent US 5,762,903 is unstable which induces rapid release or leakage of the radioactivity to the surrounding tissues or to other organs.

WO 2023/148351A1 discloses a pharmaceutical formulation of a pharmaceutically active protein comprising (i) an effective amount of a protein or a combination of proteins (antibodies), (ii) a chitosan comprising between 90 and 100

mol% of D-glucosamine and between 0 and 15% of N-acetyl-D-glucosamine (Mw = 2,583.105 g/mol, Mn = 1 ,323.105 g/mol respectively), and (iii) at least one statistic polysaccharide B comprising D-glucosamine, N-acetyl-D-glucosamine and at least one N-DOTAGA-D-glucosamine unit (15 ± 0.5%). The pharmaceutical formulation of the disclosure can easily be injected subcutaneously in a tissue due to its rheological properties while being able to forms, *in vivo,* a biodegradable hydrogel that entraps the proteins and enables their controlled and prolonged release in the tissue. The pharmaceutical formulation of the disclosure is preferably under the form of a liquid formulation, more preferably of a liquid injectable formulation.

In this context, the invention aims to satisfy at least one of the following objectives.

An objective of the invention is to provide new systems for preparing injectable, biocompatible and biodegradable hydrogels or compositions becoming hydrogel under physiological conditions which are capable of secure and controlled release of therapeutically active radioactivity after injection.

An objective of the invention is to provide new systems for preparing injectable, biocompatible and biodegradable hydrogels or compositions becoming hydrogel under physiological conditions which are capable of secure and controlled release of therapeutically active radioactivity, allowing safe and efficient brachytherapy, with a high radioactivity at the site of the tumor or of the excised tumor, for an elimination of all remaining cancer cells and without detrimental leakage or release of the radioactivity to the surrounding tissues or to other organs.

An objective of the invention is to provide new systems for preparing injectable, biocompatible and biodegradable hydrogels or compositions becoming hydrogel under physiological conditions which are capable of secure and controlled release of therapeutically active radioactivity, which do not impair the mechanical properties of the hydrogel as well as the rheological properties of pharmaceutical compositions thereof.

An objective of the invention is to provide new systems for preparing injectable, biocompatible and biodegradable hydrogels or compositions becoming hydrogel under physiological conditions which are capable of secure and controlled release of therapeutically active radioactivity, which biodegradability kinetic is adapted to the internal radiation therapy, for instance hydrogels half-life time longer than that of radionuclide, and that it can be decomposed and excreted after decay of radionuclide.

An objective of the invention is to provide new systems for preparing injectable, biocompatible and biodegradable hydrogels or compositions becoming hydrogel under physiological conditions which are capable of secure and controlled release of therapeutically active radioactivity, and which can fit closely to the shape of the *in vivo* tumors excised cavities they are intended to fill.

An objective of the invention is to provide new systems for preparing injectable, biocompatible and biodegradable hydrogels or compositions becoming hydrogel under physiological conditions which have a high affinity with radionuclides *in vivo* and *in vitro,* that it can be evenly distributed in the site of the tumor or of the excised tumor, without inflammation.

An objective of the invention is to provide new pharmaceutical compositions, pharmaceutical radioactive hydrogels and related systems according to at least one of the above objectives.

An objective of the invention is to provide new pharmaceutical compositions according to at least one of the above objectives, which compositions having rheology properties adapted to their injectability.

An objective of the invention is to provide new pharmaceutical compositions according to at least one of the above objectives, which compositions which rheology properties are not impaired by the linkage of therapeutically active radionuclides.

An objective of the invention is to provide hydrogels prepared by means of the systems or compositions thereof, according to at least one of the above objectives.

An objective of the invention is to provide a simple and efficient method for preparing pharmaceutical compositions according to at least one of the above objectives.

An objective of the invention is to provide safer and easier methods of treating a solid cancer by brachytherapy in a subject in need thereof, with less side effects and less invasive manipulations to be underwent by said subject.

## SUMMARY

The inventors have designed a new system including a new injectable pharmaceutical composition which can be a hydrogel or a liquid composition allowing the formation of a pharmaceutical radioactive hydrogel in physiological conditions, such composition comprising (i) at least one hydrophilic polymer, for instance a polysaccharide like a chitosan-derived polymer or hyaluronic acid-derived polymer, bearing at least one chelating group and (ii) at least one therapeutical radionuclide, wherein said therapeutical radionuclide is chelated to said chelating group. The composition is or becomes a hydrogel that can snugly fulfill the tissues and/or snugly fit the shape of a surgical excision cavity once injected into the tumor or surgical cavity. The entrapped therapeutical radionuclide releases a high and localized dose of radioactivity at the tumor site with no or very limited leakage or release to surrounding and/or distant tissues.

Furthermore, the hydrogel of the disclosure being biocompatible and biodegradable, it can be safely injected into the tumor of a subject in need thereof without need offurther surgery to remove it. Finally, as described in more details

below, the chemical and physical properties of the composition of the disclosure have several advantages compared to the solutions described in the prior art: they allow perfect fit of the radioactive source within the composition which gels in physiological conditions, but also adapting the dose of radioactivity to be released in the tumor site, and are particularly well-suited for cavities following the surgical resection of some tumor volume.

A first object of the disclosure relates to a system for preparing a pharmaceutical radioactive hydrogel comprising:

E.1. At least one hydrophilic polymer E.1. bearing at least one chelating group, said polymer having ability to become or remain a hydrogel under physiological conditions,
E.2. At least one therapeutical radionuclide E.2. chelatable with said chelating group, preferably under the form of a liquid with soluble radioactive cations, and more preferably of an aqueous solution.

In one embodiment of the disclosure, said hydrophilic polymer is a polysaccharide E.1.1. derived from chitosan of formula (I):

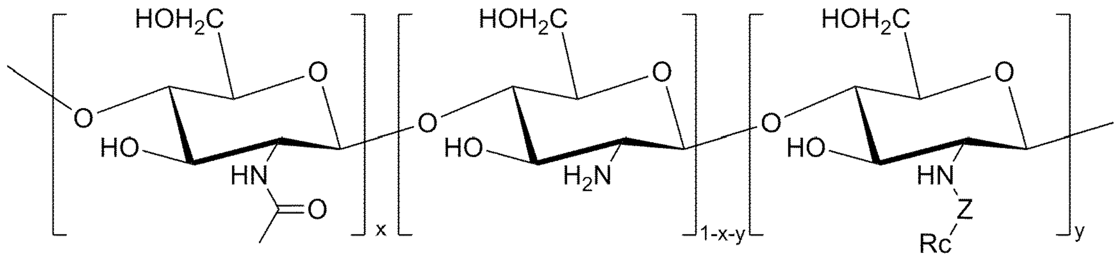
(I)

wherein:

- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- x is comprised between 0 and 0.1, preferably between 0.005 and 0.09, more preferably between 0.02 and 0.08,
- y is comprised between 0.01 and 0.4, particularly between 0.05 and 0.35, preferably between 0.08 and 0.3, more preferably 0.1 and 0.25, even more preferably between 0.1 and 0.20,
- y/x is comprised between 0.2 and 50, particularly between 0.5 and 45, preferably between 0.8 and 40, more preferably between 1 and 35, even more preferably between 1.5 and 30.
- x + y is comprised between 0.1 and 0.5, particularly between 0.2 and 0.45, preferably between 0.2 and 0.4.

and optionally further comprises at least one chitosan E.1.1.1.

In another embodiment, said hydrophilic polymer is a polysaccharide E.1.2. derived from hyaluronic acid of formula (II):

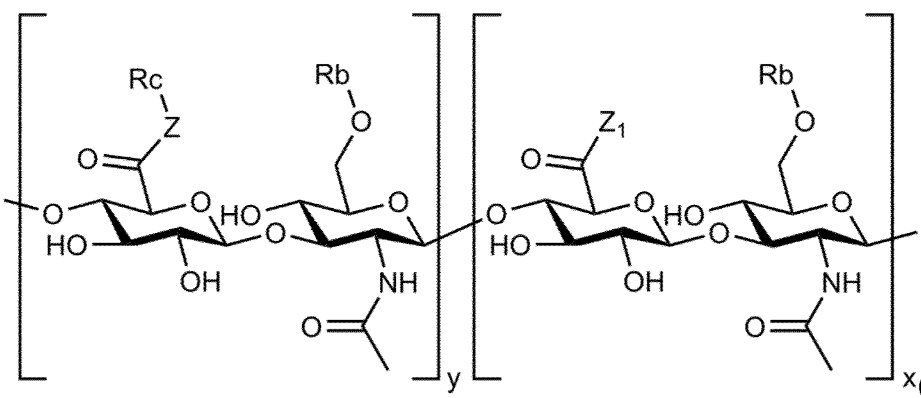
(II)

wherein:

- Rb is a crosslinking group,
- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- $Z_1$ a group comprising an hydroxyl function,
- x is comprised between 0.6 and 0.95, preferably between 0.7 and 0.85, more preferably between 0.75 and 0.85,
- y is comprised between 0.05 and 0.4, preferably between 0.15 and 0.3, more preferably 0.15 and 0.25.

A second object of the disclosure relates to a pharmaceutical composition E.3. as part of the system for preparing a pharmaceutical radioactive hydrogel of the disclosure comprising said hydrophilic polymer E.1. including said therapeutical radionuclide E.2. chelated with the chelating group Rc contained in E.1., said pharmaceutical composition being injectable.

A third object of the disclosure relates to a pharmaceutical radioactive hydrogel susceptible to be obtained from the system or the pharmaceutical composition E.3. of the disclosure under physiological conditions.

A fourth object of the disclosure relates to a pharmaceutical composition E.3. for use as a drug, in particular for use in the treatment of solid cancer, preferably by brachytherapy. The disclosure also relates to associated methods of treatment.

A fifth object of the disclosure relates to a method for preparing a pharmaceutical composition E.3. of the disclosure comprising the following steps:

(i) Implementing the hydrophilic polymer E.1. of the disclosure;
(ii) Implementing the therapeutical radionuclide E.2. of the disclosure;
(iii) Mixing the hydrophilic polymer E.1. with the therapeutical radionuclide E.2., allowing the chelation of the therapeutical radionuclide E.2. with the chelating group(s) present in the hydrophilic polymer E.1.;

wherein such aqueous liquid allows formation of a hydrogel, particularly under physiological conditions, e.g. in contact with human tissues.

## LEGEND OF DRAWINGS

**Figure 1.** Measurement of the radioactivity in compositions E.3. with different radionuclides. Figure 1A. Gallium-68 radiolabeled composition E.3. in DPBS medium (n=1). Figure 1B. Yttrium-90 radiolabeled composition E.3. in CSF medium (n=1). Figure 1C. Fluor-18 radiolabeled composition E.3. in DPBS medium (n=1).

**Figure 2.** Positron emission tomography (PET) image coupled with computed tomography (CT) of a rat brain 1 hour after the deposition of 1 MBq of the Yttrium-90 radiolabeled composition E.3. in the cerebral cortex.

**Figure 3.** dosimetry of different sources of radioactivity: 1/ control (no radioactivity), 2/Gliasite® source, 3/ Gammatile® source, 4/ composition E.3. of the disclosure.

**Figure 4.** Gelation of a composition E.3. comprising a polysaccharide E.1.1. having DOTAGA as Rc group and a chitosan E.1.1.1. at a ratio E.1.1. : E.1.1.1. of 2:1. The figure shows the gelation time and evolution of elasticity and damping factor tan(δ) over time.

## DETAILED DESCRIPTION

### *SYSTEM FOR PREPARING A PHARMACEUTICAL RADIOACTIVE HYDROGEL*

A first aspect of the disclosure relates to a system for preparing a pharmaceutical radioactive hydrogel comprising:

**E.1.** at least one hydrophilic polymer E.1. bearing at least one chelating group,
**E.2.** at least one therapeutical radionuclide E.2. chelatable with said chelating group, preferably a radioactive cation.

Advantageously, said system allows preparation of a pharmaceutical radioactive hydrogel of the disclosure as

described below. Particularly, the pharmaceutical radioactive hydrogel of the disclosure has specific rheological properties suitable to the use of the hydrogel for treatment of solid cancers, particularly by brachytherapy. More specifically, said system is suitable for preparation of a pharmaceutical radioactive hydrogel having, under physiological conditions, an elastic modulus G' between 20 Pa and 3000 Pa and a damping factor tan(δ) between 0,01 and 1.

**Hydrophilic polymer E.1.**

The term "hydrophilic polymer" has its general meaning in the art and means e.g. herein polymer which dissolve in, or are swollen by water. It particularly encompasses polymers of natural origin.

The hydrophilic polymer of the disclosure shall be biocompatible. So, as used herein, "hydrophilic polymer" means any hydrophilic polymer compatible with use in human tissues as typically used in medical applications. It particularly, without limitation, encompasses hyaluronic acid, starch, cellulose, alginate, polyethylene glycol, chitosan or a derivative thereof.

According to the present disclosure, the expression "biocompatible", referring to the *in situ* hydrogel or the injected pharmaceutical composition, means a hydrogel or composition comprising such polymer having the ability not to degrade the biological environment it is placed into. In particular, the hydrogel produces little or no inflammatory reactions even during a prolonged contact with biological environment.

Advantageously, the hydrophilic polymer E.1. is in a form of a liquid, particularly an aqueous liquid, preferably a solution.

The hydrophilic polymer E.1. bears at least one chelating group Rc linked to the polymeric chain by a linker Z.

Optionally, the hydrophilic polymer E.1. may further bear a crosslinking group Rb.

*Polysaccharides of the disclosure*

In one embodiment, the hydrophilic polymer E.1. comprises a polysaccharide chain derived from chitosan.

In a specific embodiment, the hydrophilic polymer E.1. comprises a polysaccharide derived from chitosan E.1.1. of formula (I):

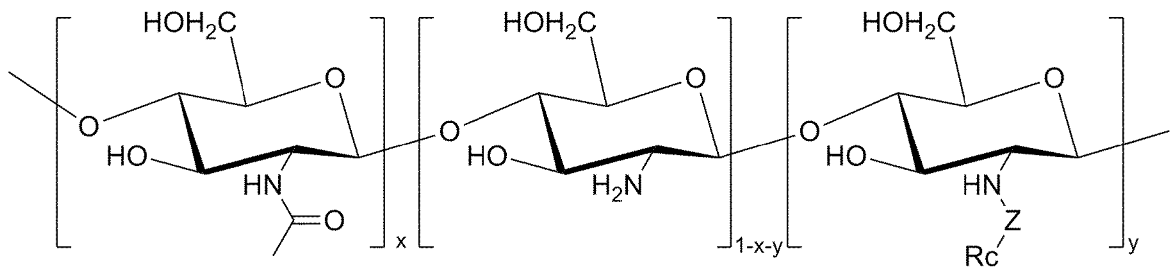

(I)

wherein:

- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- x is comprised between 0 and 0.1, preferably between 0.005 and 0.09, more preferably between 0.02 and 0.08,
- y is comprised between 0.01 and 0.4, particularly between 0.05 and 0.35, preferably between 0.08 and 0.3, more preferably 0.1 and 0.25, even more preferably between 0.1 and 0.20,
- y/x is comprised between 0.2 and 50, particularly between 0.5 and 45, preferably between 0.8 and 40, more preferably between 1 and 35, even more preferably between 1.5 and 30.
- x + y is comprised between 0.1 and 0.5, particularly between 0.2 and 0.45, preferably between 0.2 and 0.4.

Advantageously, the polysaccharide E.1.1. of formula (I) is a statistical polysaccharide.

Advantageously, the polysaccharide E.1.1. of formula (I) has a mean molecular weight between 100 kDa and 1000 kDa, more advantageously between 200 kDa and 750 kDa, even more advantageously between 250 kDa and 500 kDa and most advantageously between 300 kDa and 400 kDa. According to the present disclosure, the mean molecular weight Mw of polysaccharides such as polysaccharide E.1.1. (and more generally chitosan, including chitosan E.1.1.1. as described below) may be measured by steric exclusion chromatography, the method being described in "Physico-chemical studies of the gelation of chitosan in a hydroalcoholic medium" A. MONTEMBAULT, C. VITON, A. DOMARD,

Biomaterials, 26(8), 933-943, 2005.

In a specific embodiment of the disclosure, the hydrophilic polymer E.1. comprising a polysaccharide derived from chitosan, particularly a polysaccharide E.1.1. of formula (I) further comprises at least one chitosan E.1.1.1..

According to the present disclosure, "chitosan" means a natural polymer of polysaccharide type of D-glucosamine (GlcN) or co-polysaccharide type, consisting of a random distribution (statistic copolysaccharide) or not (blocks copolysaccharide) of D-glucosamine and N-acetyl-D-glucosamine (GlcNAc), linked by glycosidic bonds of β(1->4) type.

In a more specific embodiment wherein the hydrophilic polymer E.1. comprises a chitosan E.1.1.1. in association with the polysaccharide E.1.1., the weight ratio between the polysaccharide E.1.1 and chitosan E.1.1.1. (E.1.1. : E.1.1.1.) within the hydrophilic polymer E.1. ranges between 5:1 and 1:5, particularly between 3:1 and 1:3, preferably between 3:1 and 1:1. Typically, the polysaccharide E.1.1. is present in a ratio E.1.1. : E.1.1.1. of about 2:1.

Advantageously, the proportion of acetylation of chitosan E.1.1.1. is less than 40%, preferably less than 10%, for example between 0% and 10%. The acetylation of chitosan E.1.1.1., i.e. the proportion of N-acetyl-D-glucosamine, is calculated by using $H^1$NMR, following the Hirai's methodology (A. HIRAI, H ODANI, A. NAKAJIMA, Polymer Bulletin, 26 (1), 87-94, 1991).

According to the present disclosure, crystallinity ratio represents the proportion of crystalline material. The crystallinity ratio in chitosan hydrogels significantly influences their stability and mechanical properties. High crystallinity typically enhances mechanical strength and stability due to increased intermolecular interactions and a more ordered polymer structure. Conversely, low crystallinity can lead to reduced mechanical integrity and stability. Regarding polysaccharides, the crystallinity ratio is often determined using X diffraction (Alexander, L. E., 'X-ray Diffraction Methods in Polymer Science', Wiley-Interscience, New York, 1969, p. 137). Chitosan E.1.1.1. promotes the formation of crystallites responsible for the gel state of the materials in contact with body fluids. Advantageously, the degree of crystallinity based on chitosan E.1.1.1. can be between 10 and 25%. The chitosan E.1.1.1. crystallites act as physical cross-linking nodes, essential for gel formation. The size of the crystallites may be less than 20nm, for example between 1 and 20 nm. Advantageously, chitosan E.1.1.1. has an average molecular weight between 100 kg/mol and 1000 kg/mol, preferably between 200 kg/mol and 700 kg/mol.

In another embodiment, the hydrophilic polymer E.1. of the disclosure comprises (i) a hydrophilic polymer which is a polysaccharide chain derived from hyaluronic acid.

In a specific embodiment, the hydrophilic polymer E.1. comprises a polysaccharide derived from hyaluronic acid E.1.2. of formula (II):

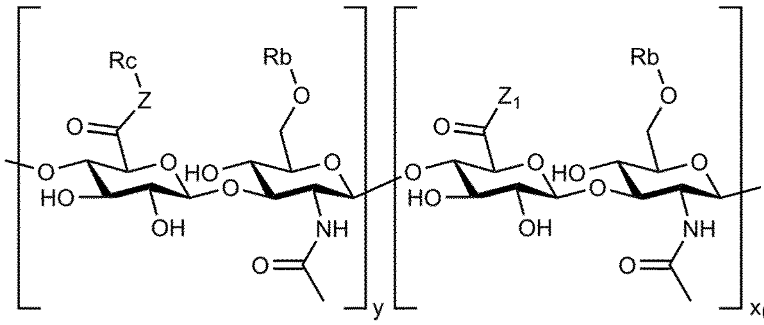

(II)

wherein:

- Rb is a crosslinking group,
- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- $Z_1$ a group comprising a hydroxyl function,
- x is comprised between 0.6 and 0.95, preferably between 0.7 and 0.85, more preferably between 0.75 and 0.85,
- y is comprised between 0.05 and 0.4, preferably between 0.15 and 0.3, more preferably between 0.15 and 0.25.

Advantageously, the polysaccharide E.1.2. hydrophilic polymer has a mean molecular weight between 200 kDa and 5000 kDa, more advantageously between 500 kDa and 7000 kDa, even more advantageously between 700 kDa and 5000 kDa and most advantageously between 1500 kDa and 3500 kDa. The molecular weight of hydrophilic polymer chain is measured by Dynamic Light Scatetering (DLS) or Size Exclusion Chromatography (SEC), in particular Size-Exclusion Chromatography coupled with Multi-Angle Light Scattering (SEC-MALS).

A skilled man in the art will be able to adapt the crosslinking properties of the polysaccharide E.1.2 to obtain the hydrogel of the disclosure (particularly in terms of visco-elasticity properties of the pharmaceutical radioactive hydrogel of the disclosure).

*Chelating group in the hydrophilic polymer*

As described above, the hydrophilic polymer E.1. bears at least one chelating group Rc.

The at least one chelating groups grafted to the hydrophilic polymer E.1., particularly the polysaccharide E.1.1. or E.1.2., may be selected from DOTA (1,4,7,10-tetraazacyclododecane-N,N',N",N‴-tetracetic acid), NOTA (1,4,7-triazacyclononane-1,4,7-triacetic acid), NODAGA (1,4,7-triazacyclononane-1-glutaric acid-4,7- diacetic acid), DOTAGA (2-(4,7,10-tris(carboxymethyl)-1,4,7,10-tetraazacyclododecan-1-yl)pentanedioic acid), DOTAM (1,4,7,10-tetrakis(carbamoylmethyl)-1,4,7,10 tetraazacyclododecane), NOTAM (1,4,7-tetrakis(carbamoylmethyl)-1,4,7-triazacyclononane), DOTP (1,4,7,10-tetraazacyclododecane 1,4,7,10-tetrakis(methylene phosphonate), NOTP (1,4,7-tetrakis(methylene phosphonate)-1,4,7-triazacyclononane), TETA (1,4,8,11-tetraazacyclotetradecane-N,N',N",N‴-tetraacetic acid), TETAM (1,4,8,11-tetraazacyclotetradecane-N,N',N",N"'-tetrakis(carbamoyl methyl), DTPA (diethylene triaminopentaacetic acid), EDTA (N,N'-(Ethane-1,2-diyl)bis[N-(carboxymethyl)glycine]) and DFO (deferoxamine).

Particularly, the chelating group Rc grafted to the hydrophilic polymer E.1. is selected from the group consisting of DOTAGA, DFO, DOTAM and DTPA.

Preferably, the chelating group Rc is DOTAGA.

Each chelating group can comprise one or more coordination sites. Preferably, coordination sites are a nitrogen or an oxygen atom. Advantageously, each chelating group comprises between 4 and 8 coordination sites, more advantageously between 6 and 8 coordination sites and even more advantageously each of chelating group comprises 8 coordination sites.

In the present disclosure, a coordination site means a unique function able to chelate a metal. For example, an amine function represents one coordination site by forming a dative bond between the nitrogen of the amine and a metal and a hydroxamic acid function also represents one coordination site by forming a dative bond between the oxygen of the carbonyl moiety and a metal and by a covalent bond between the oxygen of the N-oxyde moiety and the very same metal, the coordination site forming a 5 links ring.

*Linker Z in the hydrophilic polymer*

As mentioned above, the hydrophilic polymer E.1., particularly the polysaccharide E.1.1. of formula (I) or the polysaccharide E.1.2. of formula (II) comprises at least one linker Z.

Z is a linker being a single bond, a hydrocarbonated chain comprising between 1 and 12 carbon atoms, said chain being linear or branched, saturated or unsaturated and optionally comprising one or more heteroatoms preferably selected from nitrogen, oxygen and sulfur.

Choice of Z essentially depends on chelating group Rc. Indeed, notably for steric reasons, chelating group Rc could be more or less close to the 6 links ring.

In some embodiments, Z is selected from the group consisting of a single bond, a hydrocarbonated chain comprising between 1 and 12 carbon atoms, said chain being linear or branched, saturated or unsaturated and optionally comprising one or more heteroatoms preferably selected from nitrogen, oxygen and sulfur, an alkyl chain comprising between 1 and 12 carbon atoms, said chain being linear or branched, and an alkenyl chain comprising between 2 and 12 carbon atoms, said chain being linear or branched, said alkyl and alkenyl chains could be interrupted by one or more aryl groups in $C_6$-$C_{10}$, and/or one or more heteroatoms or groups selected from the group consisting of-O-, -S-, -C(O)-, -NR'-, -C(O)NR'-, -NR'-C(O)-, -NR'-C(O)-NR'-, -NR'-C(O)-O-, -O-C(O)NR', -C(S)NR'-, -NR'-C(S)-, -NR'-C(S)-NR' said alkyl and alkenyl chains could be substituted by one or more of the atoms or groups selected from the group consisting of -OR', -COOR', -SR', $-NR'_2$, each R' being independently H or an alkyl in $C_1$-$C_6$.

Advantageously, Z is selected from the group consisting of a single bond, an alkyl chain comprising between 1 and 12 carbon atoms, said chain being linear or branched, said alkyl chain could be interrupted by one or more aryl groups in $C_6$-$C_{10}$, and/or one or more heteroatoms or groups selected from the group consisting of-O-, -S-, -C(O)-, -NR'-, -C(O) NR'-, -NR'-C(O)-, -C(S)NR'-, -NR'-C(S)-, -NR'-C(S)-NR' each R' being independently H or an alkyl in $C_1$-$C_6$.

In a particular embodiment, Z is an alkyl chain comprising between 1 and 12 carbon atoms.

*Crosslinking group in the hydrophilic polymer*

Optionally, the hydrophilic polymer E.1. comprises a crosslinking group Rb.

Advantageously, said crosslinking group Rb is present if the hydrophilic polymer is hyaluronic acid, particularly a polysaccharide E.1.2. of formula (II).

The term "crosslinking group" refers to a bond or a short sequence of bonds that links one polymer chain to another, under the form of covalent bonds or ionic bonds. It particularly encompasses, without limitations, 1,4-butanediol diglycidyl ether (BDDE), poly (ethylene glycol) diglycidyl ether (PEGDE), divinyl sulfone (DVS), 1-ethyl-3-(3 dimethylaminopropyl) carbodiimide (EDC), 1,2,7,8-diepoxyoctane et glutaraldehyde (GA).

*Process for preparing the hydrophilic polymer of E.1.*

The hydrophilic polymer contained in E.1. could be obtained as follows:

- Step 1 : solubilizing a hydrophilic polymer,
- Step 2 : grafting said hydrophilic polymer with a linker,
- Step 3: grating the chelating group to the modified polymer obtained at step 2,
- Step 4: optionally, crosslinking of the modified polymer obtained after step 3 and then dialysing the obtained crosslinked modified polymer.

Step 4 is mandatory in the case the hydrophilic polymer is a polysaccharide derived from hyaluronic acid and optional in the case the hydrophilic polymer is a polysaccharide derived from chitosan. Typically, dialysis in step 4 is carried out against a buffer with physiological pH and osmolarity and allows elimination of crosslinking agent.

In one embodiment, a polysaccharide E.1.1. of the disclosure could be obtained with a process comprising the three following successive steps:

- Step 1 : solubilizing a chitosan in an acidic solution at a pH between 4 and 5;
- Step 2 : partial acetylation of amine functions of the chitosan solubilized in step 1;
- Step 3 : functionalization of at least a part of the remaining amine functions after step 2.

Step 3 could be subdivided in several under steps, notably when Z is a hydrocarbonated chain as defined above.

In embodiments where Z is a hydrocarbonated chain as defined above, step 3 may comprise an under step 3-1 consisting in grafting said hydrocarbonated chain on at least a part of the remaining amine functions after step 2, and then an under step 3-2 consisting in grafting of Rc, $Rc_1$ and/or $Rc_2$. Alternatively, step 3 does not comprise an under step. In this alternative, said hydrocarbonated chain is coupled with Rc, said step 3 is, in this case, performed with a molecule comprising Rc and said hydrocarbonated chain.

In an alternative manner, the polysaccharide E.1.1. could be obtained starting from a chitosan having the suitable number of N-acetyl-D-glucosamine units. In this alternative, the above step 2 has not to be performed, thus, in this alternative the process for obtaining polysaccharide E.1.1. comprises at least the two following successive steps:

- Step 1b: solubilizing a chitosan, comprising N-acetyl-D-glucosamine units, in an acidic solution at a pH between 4 and 5;
- Step 2b : functionalization of at least a part of the amine functions of said chitosan, comprising N-acetyl-D-glucosamine units solubilized at step 1.

In the same manner as above for said step 3, said step 2b could be subdivided in several under steps, notably when Z is a hydrocarbonated chain as defined above.

In embodiments where Z is a hydrocarbonated chain as defined above, step 2b may comprise an under step 2b-1 consisting in grafting said hydrocarbonated chain on at least a part of the amine functions, and then an under step 2b-2 consisting in grafting of Rc, $Rc_1$ and/or $Rc_2$ on said hydrocarbonated chain. Alternatively, step 2b does not comprise an under step. In this alternative, said hydrocarbonated chain is coupled with Rc, $Rc_1$ and $Rc_2$, said step 2b is, in this case, performed with a molecule comprising Rc, $Rc_1$ or and said hydrocarbonated chain.

In another embodiment, a polysaccharide E.1.2. of the disclosure could be obtained with a process as described in Example 5 of the present disclosure.

## Therapeutical radionuclide E.2.

As described above, the system of the disclosure further comprises at least one therapeutical radionuclide E.2.

As used herein, the term "therapeutical radionuclide" has its general meaning in the art and refers to a radionuclide that is usable for therapeutical purposes which presents the following main characteristics:

- it emits a particle, preferably an electron (directly or after electronic capture) or an alpha particle,
- its daughter can emit a deexcitation gamma ray with an energy of a few dozen to hundred keV, ideally between 100 and

200 keV, ideally 140 keV.

- the radionuclide physical half-life range for 10h ($^{212}Pb$) up to 10 days ($^{225}Ac$), ideally between 2 and 5 days.

According to the invention, said therapeutical radionuclide is a cation and may be chosen, without limitation, from the group Bismuth-213, Cesium-131, Holmium-166, Iridium-192, Lutetium-177, Mercury-197, Rhenium-186, Rhenium-188, Yttrium-90, Terbium-155, Terbium-149, Terbium-161, Astatine-211, Actinium-225, Lead-212, Radium-223, Copper-64, Copper-67.

The therapeutical radionuclide E.2. is chelatable with the chelating group Rc present in the hydrophilic polymer E.1.. It forms a non-covalent bond with the functional groups of the chelating group Rc, particularly the chelating group Rc of formulas (I) and (II) in the specific embodiments of a hydrophilic polymer E.1.1. of E.1.2..

In one embodiment, the therapeutical radionuclide E.2. is selected from the group Bismuth-213, Cesium-131, Holmium-166, Iridium-192, Lutetium-177, Mercury-197, Rhenium-186, Rhenium-188, Yttrium-90, Terbium-155, Terbium-149, Terbium-161, Astatine-211, Actinium-225, Lead-212, Radium-223, Copper-64, Copper-67, particularly Yttrium-90, Lutetium-177 or Actinium-225. Preferably, the therapeutical radionuclide E.2. is Yttrium-90.

Advantageously, the therapeutical radionuclide E.2. is in the form of a liquid comprising soluble radioactive cations, particularly an aqueous solution.

In one embodiment, the therapeutical radionuclide E.2. further comprises a pH adjuster.

As used herein, a "pH adjuster" is a chemical that is added to a solution to adjust a pH value of the solution and to thereby achieve a desired performance. Controlling the pH can be performed by adding a pH adjuster to the formulation. Examples of pH adjusters include commonly used acids and bases, buffers and mixtures of acids and bases. For example, bases that can be used include AcONa, NaOH, KOH, Ca(OH)2, sodium bicarbonate, potassium carbonate, and sodium carbonate. Examples of acids that can be used include hydrochloric acid, acetic acid, citric acid, formic acid, fumaric acid, and sulfamic acid. Preferably the pH adjuster is a base, more preferably NaOH. The range of pH of the fluid can be any suitable range, such as about 2 to about 14.

Advantageously, the system of the disclosure comprises a notice of instructions for preparing the pharmaceutical composition precursor of the pharmaceutical radioactive hydrogel and/or instructions for use.

## Specific embodiment of the system of the disclosure

In a preferred embodiment, the system of the disclosure comprises:

**E.1.** A hydrophilic polymer E.1. under the form of an aqueous solution comprising:

E.1.1. at least one statistical polysaccharide E.1.1. of formula (I) having a weight-average molecular weight of between 100 kDa and 1000 kDa wherein

- Rc is DOTAGA,
- Z is a single bound,
- x is between 0.02 and 0.08, for example 0.06,
- y is between 0.10 and 0.20, for example 0,17,

E.1.2. at least one chitosan E.1.1.1. with less than 10% acetylation present in a ratio E.1.1.: E.1.1.1. of 2:1; and

**E.2.** An aqueous solution comprising Yttrium-90 radionuclide E.2. chelatable with DOTAGA of E.1.1..

In another preferred embodiment, the system of the disclosure comprises:

**E.1.** A hydrophilic polymer under the form of an aqueous solution comprising at least one polysaccharide E.1.2. of formula (II) having a weight-average molecular weight of between 5000 kDa and 7000 kDa wherein

- Rb is BDDE,
- Rc is DOTAGA,
- Z is ethylendiamine,
- Z1 is OH,
- x is between 0.75 and 0.85, for example 0,77,
- y is between 0.15 and 0.25, for example 0.23, and

**E.2.** An aqueous solution comprising Yttrium-90 radionuclide E.2. chelatable with DOTAGA of E.1.2..

***PHARMACEUTICAL COMPOSITION E.3.***

A second aspect of the disclosure relates to a pharmaceutical composition E.3. as part of the system of the disclosure comprising a hydrophilic polymer E.1. including a therapeutical radionuclide E.2. chelated with the chelating group Rc contained in E.1.. According to the disclosure, the pharmaceutical composition E.3. is injectable.

According to the present disclosure, an "injectable pharmaceutical composition" means a liquid or gel formulation having visco-elasticity properties allowing a good injectability through a gauge needle. Advantageously, the injectable pharmaceutical composition of the disclosure preferably has an ejection force up to 60N, preferably up to 50N. The term "ejection force" has its general meaning in the art and refers to the force required to eject the material from the syringe equipped with a suitable needle at a speed similar to that applied in real-life situations (ie. 10mm/min - 60mm/min). It may be measured as described in Watt RP et al., Inet J Pharm, 2019 Jan 10:554:376-386.

The term "injectability", as generally used herein, particularly refers to the injection performance of the pharmaceutical composition of the disclosure through a syringe equipped with a 18-32 gauge needle. One skilled in the art will be able to choose an adapted needle for injection of the pharmaceutical composition of the disclosure.

Advantageously, the percentage of chelation of the therapeutical radionuclide E.2. with the chelating group(s) present in E.1. is between 85 and 100%, particularly between 90 and 100%, preferably higher than 95%.

A skilled man in the art is able to choose and adapt the concentration of the therapeutical radionuclide E.2. depending on the different features of the pharmaceutical composition E.3.. Advantageously, the therapeutical radionuclide is present at a concentration that it provides a volumetric radioactivity of between 10 and 250 MBq/mL to yield the same deposited dose after 4 days (with $^{90}Y$) 3 mm away from the edge of the cavity. This range is estimated from simulations of 8.12 mL (clinical case) and 4.19 $\mu$L (preclinical case) respectively. These activity concentrations have to be compared with the order of magnitude used in targeted radiotherapy where roughly 15 to 20 GBq are injected to the patients. Activity concentration for any radionuclide can be deduced from pre-computed S-factors (expressed in $Gy.MBq^{-1}.s^{-1}$) of targeted and elimination organs for these isotopes.

According to the invention, said pharmaceutical composition E.3. may be in the form of (i) a liquid, which is a precursor of the pharmaceutical radioactive hydrogel of the disclosure or (ii) a hydrogel, i.e. an injectable pharmaceutical radioactive hydrogel of the disclosure, depending on the nature of the hydrophilic polymer E.1..

Particularly, in the specific embodiment of the hydrophilic polymer E.1. comprising a polysaccharide E.1.1. (optionally further comprising a chitosan E.1.1.1.), the pharmaceutical composition E.3. is a liquid. Advantageously, said liquid pharmaceutical composition is an aqueous liquid, preferably an aqueous solution. In this specific embodiment, the pharmaceutical composition of the disclosure is a precursor of the pharmaceutical radioactive hydrogel of the disclosure and becomes a radioactive hydrogel of the disclosure under physiological conditions after *in vivo* injection. Particularly, the liquid pharmaceutical composition of the disclosure forms a hydrogel by a change in pH and/or osmolarity by equilibration with physiological media.

Particularly, in the other specific embodiment of the hydrophilic polymer E.1. comprising a polysaccharide E.1.2., the pharmaceutical composition E.3. is a hydrogel. In this specific embodiment, the pharmaceutical composition is injectable and remains a hydrogel *in vivo,* after injection.

According to the disclosure, the term "under physiological conditions" refers to *in vivo* osmolarity and/or pH conditions and also encompasses physio-pathological conditions. As used herein, physiological osmolarity typically means an osmolarity between 200 and 600 mOsm/L, more preferably between 250 and 450 mOsm/L, typically of approximately 300mOsm/L and physiological pH means a pH between 6 and 8, particularly between 6.8 and 7.6, typically of approximately 7.4.

According to the present disclosure, the osmolarity (also known as osmotic concentration) refers to the solute concentration per unit volume of solution. Osmolarity is similar to molarity but includes the total number of moles of dissolved species in solution. Osmolarity is defined as the number of osmoles (Osm) of solute per litre (L) of solution (osmol/L or Osm/L). An osmole is the amount of substance, which must be dissolved in order to produce an Avogadro's number of particles ($6.0221 \times 10^{23}$). For substances, which do not dissociate, the molarity and the osmolarity will be the same, whereas for substances that are ionized the osmolarity will be the molarity multiplied by the number of dissociated parts, eg. for sodium chloride the osmolarity will be doubled. Osmolality is the number of osmoles of solute per kilogram of solvent. Physiological osmolarity is typically in the range of about 280 mOsm/L to about 310 mOsm/L, typically is about 300 mOsm/L.

The osmolarity of the pharmaceutical composition of the disclosure is typically measured using an osmometer, such as a Löser Micro Osmometer MOD200 Plus from Camlab. Prior to measurement, zero is set using 50 $\mu$L of distilled water, and the instrument is then calibrated against 25$\mu$L of a 300mOsm/kg water standard. Samples are measured by taking an identical volume of 25 $\mu$L frozen to -6.0 °C. Alternatively, zero is set using 15 $\mu$L of distilled water, and the instrument is then calibrated against 15$\mu$L of a 300mOsm/kg water standard. Samples are measured by taking an identical volume of 15 $\mu$L frozen to -6.2 °C.

The pH of the pharmaceutical composition of the disclosure is measured according to methods known to those

skilled in the art, for example using a pH meter such as a Mettler Toledo SevenCompact S210 pH meter.

*Pharmaceutical compositions E.3.1. comprising a polysaccharide E.1.1.*

In the specific embodiment of a hydrophilic polymer E.1. comprising a polysaccharide E.1.1. (and optionally a chitosan E.1.1.1.), the pharmaceutical composition comprising such polysaccharide E.1.1., hereinafter called "pharmaceutical composition E.3.1.", is a liquid before injection and a precursor of a pharmaceutical radioactive hydrogel of the invention and preferably has a pH and/or an osmolarity close, but different from the physiological conditions, and in particular lower than the physiological conditions.

Advantageously, the pharmaceutical composition E.3.1. has a pH comprised between 5.0 and 6.5, preferably between 5.0 and 6.2, more preferably between 5.5 and 6.0.

As described above, the pharmaceutical composition E.3.1. is a precursor of the hydrogel of the disclosure and will become such a hydrogel after *in vivo* injection, in contact with body fluids and/or tissues.

In a particular embodiment related to the pharmaceutical composition E.3.1., the hydrophilic polymer, particularly the polysaccharide E.1.1 and optionally chitosan E.1.1.1., is preferably present in the liquid pharmaceutical composition in dissolved form, meaning that at least 90%, preferably at least 95 % of the mixture is in dissolved form, % expressed by weight of mixture in dissolved form, relative to the total weight of mixture in the liquid pharmaceutical composition.

The presence of the mixture of polysaccharides, particularly E.1.1. and E.1.1.1. in dissolved form in the liquid pharmaceutical composition E.3.1. enables the liquid pharmaceutical composition to be under the form of a viscous solution sufficiently liquid to be injected via a wide range of needles commonly used in the medical field.

A skilled man in the art is able to adapt the features of the hydrophilic polymer E.1.1. in order to obtain an injectable pharmaceutical composition E.3.1 according to the disclosure.

Particularly, the pharmaceutical composition E.3.1. has a viscosity comprised between 1 and 100 Pa.s., more particularly between 6 and 60 Pa.s, preferably between 10 and 30 Pa.s as measured at room temperature by rotational rheometry in plane cone geometry at a shear rate of between 0.01 and 0.001 $s^{-1}$, for example of 0.001 $s^{-1}$ or0.01 $s^{-1}$, preferably of 0.01 $s^{-1}$.

The term "viscosity" refers to the resistance of a substance (typically a liquid) to flow. Viscosity is related to the concept of shear force; it can be understood as the effect of different layers of the fluid exerting shearing force on each other, or on other surfaces, as they move against each other. The units of viscosity are $Ns/m^2$, known as Pascal-seconds (Pa.s).

The viscosity of the pharmaceutical composition E.3.1. is typically measured by rotational rheometry in plane cone geometry using a viscometer with imposed deformation, such as an Advanced Rheometer AR2000 from TA instruments. Briefly, the fluid is subjected to shear between two surfaces, one fixed and one rotating around its axis. In practice, these two surfaces are commonly a cone and a plane. The shear gradient is determined by the geometry of the surface on the one hand, and the speed of rotation on the other hand. The shear stress is calculated from the measurement of the torque transmitted by the sample to be characterized. A detailed description of this method can be found in El Kissi et al, Rheology, 10,13-39 (2006).

*Pharmaceutical compositions E.3.2. comprising a polysaccharide E.1.2.*

In the specific embodiment of a hydrophilic polymer E.1. comprising a polysaccharide E.1.2., the pharmaceutical composition of the disclosure, hereinafter called "pharmaceutical composition E.3.2.", is already in the form of a hydrogel before injection, i.e. a pharmaceutical radioactive hydrogel of the invention, and preferably has a pH and/or an osmolarity close from the physiological conditions.

A skilled man in the art is able to adapt the features of the hydrophilic polymer E.1.2. in order to obtain an injectable hydrogel pharmaceutical composition E.3.2. according to the disclosure.

**Contents of the different components**

The content of hydrophilic polymer E.1. in the pharmaceutical composition of the disclosure is typically comprised between 10 and 100 mg/g.

Particularly, in the specific embodiment of the pharmaceutical composition E.3.1., the content of polymer E.1. (comprising polysaccharide E.1.1 and optionally chitosan E.1.1.1) in the pharmaceutical composition of the disclosure is preferably comprised between 10 and 100 mg/g, preferably between 25 and 75 mg/g, typically 50mg/g of polysaccharide E.1.1.

In another embodiment, the content of polysaccharide E.1.2. in the pharmaceutical composition E.3.2. of the disclosure is preferably comprised between 5 and 30 mg/g, preferably between 15 and 25 mg/g, typically 20 mg/g of polysaccharide E.1.2.

Variation of the different properties of the components of the composition will allow adapting the dose of radioactivity to be administered. Indeed, by adjusting the activity concentration in E.2., by adding the suitable volume of solvent, the resulting activity concentration in E.3. will be also adjusted. This modulation will subsequently adapt the dose rate at the edge of the composition.

**Pharmaceutical ingredients**

The pharmaceutical composition E.3. of the disclosure comprises a pharmaceutically acceptable carrier and/or pharmaceutically acceptable excipients.
The pharmaceutically acceptable excipient can be any of those conventionally used, and is limited only by physico-chemical considerations, such as solubility and lack of reactivity with the active compound(s).
In particular, the one or more excipient(s) can be selected from buffer and/or solvent, and/or pH adjuster.
Buffers include acetate buffer, citrate buffer and phosphate buffer. In an embodiment said buffer is acetate buffer.
In an embodiment said solvents is water for injection.
In an embodiment said pH adjuster is NaOH.
Advantageously, the pharmaceutical composition E.3. is injectable in a subject in need thereof.
The pharmaceutical composition E.3. can easily be injected in a tissue due to its rheological properties while being able to forms *in vivo* a biodegradable hydrogel that entraps the radionuclides and enables a controlled and localized radiotherapy in the tumor or close to the tumor radioactive release to distant tissues.
The pharmaceutical composition E.3., once injected into a surgical excision cavity, first fills the cavity and then forms a hydrogel that fits the shape of the cavity avoiding empty space. This allows homogeneous irradiation of the cavity margins.

***PHARMACEUTICAL RADIOACTIVE HYDROGEL***

A third aspect of the disclosure relates to a pharmaceutical radioactive hydrogel susceptible to be obtained from the system for preparing a pharmaceutical radioactive hydrogel of the invention, or from the pharmaceutical composition E.3. of the disclosure.
According to the present disclosure, the term "gel" means a nonfluidic polymeric network swollen by a solvent such as water for hydrogels.
According to the present disclosure, the expression "hydrogel" means three-dimensional network of hydrophilic polymer chains that are capable of retaining a significant amount of water or biological fluids within their structure, particularly a visco-elastic material comprising at least 60% by weight of water. The hydrogel is a physical gel. In such physical gel, crosslinking occurs through non covalent, weak, physical, reversible interactions (in opposition with covalent bonds), such as Van Der Waals interactions, hydrogen bonding and/or electrostatic interaction.
The hydrogel comprises approximately from 50% to 95% by weight of water, preferably from 50% to 80% by weight of water, relative to the total weight of hydrogel.
As described above, in a specific embodiment, the pharmaceutical composition E.3.1. (i.e. comprising a hydrophilic polymer E.1. comprising a polysaccharide E.1.1), precursor of the hydrogel, is capable of gelling *in situ* under physiological conditions, in particular at physiological pH and osmolarity, thus forming a pharmaceutical radioactive hydrogel of the disclosure.
In another specific embodiment, the pharmaceutical composition E.3.2 (i.e. comprising a hydrophilic polymer E.1. comprising a polysaccharide E.1.2.) is a pharmaceutical radioactive hydrogel of the disclosure and remains in the form of such a hydrogel after *in vivo* injection.
The hydrogel formed in the tissue is biodegradable and biocompatible. Moreover, the decay of the radionuclide enables the area of interest to be irradiated in a controlled and prolonged manner, avoiding dispersion of the radionuclide in healthy tissue.
According to the present disclosure, the expression "biodegradable", referring to the *in situ* formed hydrogel, means that the hydrogel naturally breaks down under physiological conditions, and in particular under the action of macrophages. The reactions involved during biodegradation may include hydrolysis reactions, that is to say the breaking of covalent bonds by reaction with water. These reactions may be catalyzed by the action of enzymes naturally present at the site of injection.
When injected into a cavity, the pharmaceutical composition (in the form of a liquid or a hydrogel) takes the shape of the cavity in which it is inserted into.
In an embodiment, more than 50 %, preferably more than 80 %, preferably more than 95% by weight of the hydrogel deteriorates in a period of time comprised between 10 and 100 days, preferably between 20 and 65 days, when the hydrogel is placed in physiological conditions, typically after intra tumoral or intra-surgery cavity injection in a subject.

**Visco-elasticity of the hydrogel**

The pharmaceutical radioactive hydrogel has properties suitable for the treatment of solid cancers, particularly by brachytherapy. According to the disclosure, said hydrogel has specific visco-elasticity features that allow cancer treatment by localized radioactivity without leakage of such radioactivity to surrounding or distant tissues and organs, i.e. less than 5%, ideally less than 1% of radioactivity release outside the injection site. These features particularly allow safe and efficient radiotherapy treatment with limited side effects.
The pharmaceutical radioactive hydrogel particularly has a damping factor tan(δ) of less than 1, particularly between 0,01 and 1 under physiological conditions (e.g. after *in vivo* injection).
The damping factor, also known as tan(δ) (delta tangent), is a parameter that measures energy dissipation in a viscoelastic material. It is defined as the ratio between the viscosity (dissipative part) and elasticity (reactive part) of the material, as follows:

$$\tan(\delta)=G''/G',$$

wherein:

- G' is the elastic modulus and represents the material's capacity to store energy in elastic form, indicating an elastic response (reversible deformation),
- G" is the viscous modulus and represents the material's capacity to dissipate energy in the form of heat, indicating a viscous response (irreversible deformation).

Generally, a high tan(δ) indicates high energy dissipation (more fluid or viscous properties), while a low tan(δ) suggests more elastic behavior (more solid or rigid properties). This factor is particularly useful for characterizing the texture and dynamic behavior of hydrogels in industrial applications, such as biomedical devices or cosmetics.
The pharmaceutical radioactive hydrogel particularly has an elastic modulus G' between 20 Pa and 3000 Pa under physiological conditions.
The elastic modulus of an object is defined as the slope of its stress-strain curve in the elastic deformation region; A stiffer material will have a higher elastic modulus. An elastic modulus has the form:

$$\delta = \text{stress} / \text{strain},$$

wherein:

- stress is the force causing the deformation divided by the area to which the force is applied, and
- strain is the ratio of the change in some parameter caused by the deformation to the original value of the parameter.

The damping factor and the elastic modulus of the pharmaceutical radioactive hydrogel of the disclosure are typically measured using a rheometer by methods well known in the art, for example as described in Example 4.
As explained above, the pharmaceutical radioactive hydrogel of the invention comprising a polysaccharide E.1.1. derived from chitosan gels after injection under physiological conditions.
Said hydrogel particularly has a damping factor tan(δ) of less than 1 and an elastic modulus G' between 500 Pa and 3000 Pa under physiological conditions 24 hours after injection (i.e. under physiological conditions).
As explained above, the pharmaceutical radioactive hydrogel of the invention comprising a polysaccharide E.1.2. derived from hyaluronic acid is under a hydrogel form before injection and particularly has a damping factor tan(δ) between 0.1 and 0.5 and an elastic modulus G' between 20 Pa and 200 Pa under physiological conditions.

***THERAPEUTICAL USE***

A fourth aspect of the disclosure relates to the pharmaceutical composition E.3. of the disclosure as described above for use as a drug.
The disclosure also relates to the use of the pharmaceutical composition E.3. as disclosed herein in the manufacture of a medicament, particularly for the treatment of solid cancer.
Particularly, the disclosure relates to the pharmaceutical composition E.3. of the disclosure for use in the treatment of solid cancer.
In the present disclosure, the term" treatment" encompasses curative treatment. It particularly encompasses

treatments that aim to cure a disease, slow down the progression of a disease or improve symptoms associated with a disease, but also prevent relapse of a disease.

The term "solid cancer" has its general meaning in the art and refers to cancer if any of the body organs and tissues, in opposition to blood cancer (or liquid cancer) that refer to cancer of blood cells such as leukemia, lymphoma or multiple myeloma.

As used herein, the terms "cancer" refers to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness.

The terms "solid cancer" encompasses adrenal cortical cancer, anal cancer, bile duct cancer, bladder cancer, bone cancer, brain and central nervous system cancer, particularly glioblastoma, breast cancer, cervical cancer, colorectal cancer, endometrial cancer, esophagus cancer, gallbladder cancer, gastrointestinal carcinoid tumors, sarcoma, kidney cancer, laryngeal and hypopharyngeal cancer, liver cancer, lung cancer, mesothelioma, plasmacytoma, nasal cavity and paranasal sinus cancer, nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma, salivary gland cancer, skin cancer, stomach cancer, testicular cancer, thymus cancer, thyroid cancer, vaginal cancer, vulvar cancer, and uterine cancer.

In a specific embodiment, the solid cancer is selected from liver cancer, prostate cancer, breast cancer, melanoma, glioblastoma.

In a preferred embodiment, the solid cancer is glioblastoma.

Advantageously, the pharmaceutical composition E.3. of the disclosure is for use in brachytherapy.

The term "brachytherapy" refers to a method of irradiation which involves placing radioactive sources within tissues affected by cancer, within a tumor or in a surgical excision cavity after tumor resection. This treatment is therefore targeted directly at the area which is affected by cancer. It is also called internal radiotherapy.

The disclosure also relates to a method of treating solid cancer in a subject in need thereof said method comprising administering a therapeutically effective amount of the pharmaceutical composition of the disclosure in the tumor or in the surgical excision cavity to the patient in need thereof.

In one embodiment, the pharmaceutical composition of the disclosure is administered directly in the surgical excision cavity during surgery.

The pharmaceutical composition E.3. is suitable for injection, preferably intra-tumoral injection or intra surgical excision cavity during tumor resection surgery.

According to the present disclosure, "intra-tumoral injection" means a direct injection into a tumor. Intra-tumoral injections can be considered for any tumor where the primary lesion or its metastases are accessible either percuteanously (i.e. needle-puncture of the skin) via direct injection or via specific procedures such as colonoscopy, cystoscopy, bronchoscopy, thoracoscopy, coelioscopy, or even surgery.

The injection may be administered with a 18-32-gauge needle, preferably a 20-27 gauge needle. The formulations can be administered using a small gauge needle, for example typically 21, 27, 28 29, 30 or 32 gauge. For a pharmaceutical composition E.3.1, a 21-gauge needle is for example used while for a pharmaceutical composition E.3.2., a 22-30-gauge needle is preferred.

The intra-tumoral or intra-cavity injection volume may be comprised between 1 and 20 ml, preferably between 2 and 15 ml.

***METHOD FOR PREPARING THE PHARMACEUTICAL COMPOSITION E.3.***

A fifth aspect of the disclosure relates to a method for preparing a pharmaceutical composition E.3. according to the d comprising the following steps:

(i) implementing the hydrophilic polymer E.1. of the disclosure;
(ii) implementing the therapeutical radionuclide E.2. of the disclosure;
(iii) Mixing the hydrophilic polymer E.1. with the therapeutical radionuclide E.2., allowing the chelation of the therapeutical radionuclide E.2. with the chelating group(s) present in the hydrophilic polymer E.1.;
(iv) Optionally adjusting the pH of the composition obtained in step (iii) to obtain the

pH of the pharmaceutical composition E.3. of the disclosure, as described above; wherein such composition is in the form of a hydrogel or allows formation of a hydrogel, particularly under physiological conditions, e.g. in contact with human tissues.

In a specific embodiment, the hydrophilic polymer E.1. comprises a polysaccharide chain derived from chitosan, particularly a polysaccharide E.1.1. of formula (I) as defined above and optionally further comprises a chitosan E.1.1.1..

In an embodiment, the hydrophilic polymer E.1. comprises a polysaccharide chain derived from hyaluronic acid, particularly a polysaccharide E.1.2. of formula (II) as defined above.

In a preferred embodiment, the chelating group grafted to the hydrophilic polymer E.1. is DOTAGA.

In a preferred embodiment, the therapeutical radionuclide E.2. is Yttrium-90.

The hydrophilic polymer E.1. and the therapeutical radionuclide E.2. are preferably in the form of aqueous solutions.

The components of E.1. may be obtained as described in the first aspect of the disclosure above. Such components may be dissolved in a solvent, preferably water.

The therapeutical radionuclide E.2. in form of an aqueous solution may be prepared either by eluting a generator (e.g. $^{90}$Sr - $^{90}$Y) or purchasing a pre-conditioned radiopharmaceutical precursor.

Step (iii) may be carried out by static mixer. In that embodiment, the solution of step a) is contained in a first container and the solution of step b) is contained in a second container, said containers being connected to a static mixer, to provide a system for mixing the solution of step a) and the solution of step b) by injection of the contents of one solution into the other and vice versa. Such injection may be repeated between 100 and 500 times, typically between 100 and 300 times.

The pH adjustment of step (iv) may be carried out by appropriate addition of a base or an acid, preferably of a base such as NaOH.

## EXAMPLES

The present disclosure is further illustrated by the following examples.

**EXAMPLE 1:** example of hydrophilic polymer E.1.

*Formulation of E.1. comprising a polysaccharide derived from chitosan comprising DOTAGA* as *chelating agent (chitosan@DOTAGA):* This synthesis of E.1. involves a polysaccharide obtained after chemical modification of chitosan (see below) with DOTAGA anhydride. Briefly, 60 g of chitosan were introduced into a 10 L reactor with 4 L of ultrapure water and 50 mL of acetic acid, then the mixture was placed under mechanical stirring at 500 rpm. After complete dissolution of the chitosan (3h), 4L of 1,2-propanediol was added to the medium and the mixture was kept under stirring until homogenization (2h). 120g of DOTAGA anhydride was then added and the mixture was kept under stirring overnight. The synthesis product was then purified by tangential filtration using the Sartoflow® Advanced device with a Sartocon® Slice PESU cassette (polyethersulfone membranes; cut-off: 100 kDa; filtration area: 0.1 $m^2$) according to a diafiltration-concentration model against 200L of a 0.1 M acetic acid solution, then 200L of a 5mM acetic acid solution. The degree of acetylation of polysaccharide E.1.1. is identical to that of the starting chitosan and the degree of substitution of polysaccharide E.1.1. (proportion of N-DOTAGA-D-glucosamine unit) was determined by the copper chelation method (Natuzzi et al., Nature Scientific Reports, 2021, 11, 19948) and is estimated to be 15 $\pm$ 0.5%. The synthesis of E.1. involves a non-functionalized precursor chitosan (of medical grade and animal origin. The weight and number average molar masses (Mw = 2,583.105 g/mol, Mn = 1,323.105 g/mol respectively) were determined by size exclusion chromatography coupled with refractive index and multi-angle laser light scattering measurements (Schatz et al. Biomacromolecules, 2003;4(3):641-8). The degree of acetylation (proportion of N-acetyl-D-glucosamine unit) of such crude chitosan (Chitosan) was determined by 1H NMR spectroscopy using the Hirai method (Hirai et al., Polymer Bulletin, 26, 87-94.1991) and is estimated to be 6 $\pm$ 0.5%. This chitosan can also be used as chitosan E.1.1.1. of the disclosure.

*Formulation of chitosan@DOTAGA:chitosan mixtures (polymer E.1. comprising a polysaccharide derived from chitosan comprising DOTAGA* as *chelating agent and a further chitosan E.1.1.1.)* : These mixtures were prepared at 10% or 5% w/w total polymer concentration by addition of chitosan@DOTAGA, chitosan, MilliQ water and glacial acetic acid. Chitosan@DOTAGA and chitosan amounts were adapted to reach the different ratios between the two polymers concentrations (*i.e*. chitosan@DOTAGA:chitosan 100:0 %, 83:17 %; 67:33 %, 50:50 %, 0:100 % ratios). In the same way, the quantity of acetic acid was adapted for each formulation in order to solubilize the polymers as follows: the concentration of acetic acid was 1.6% w/w for 0:100 % formulations, 0.6 % w/w for 50:50 % formulations, 0.4 % w/w for 67:33 % formulations, 0.2 % w/w for 83:17 % formulations, and no acetic acid was added for 100:0 % formulations. The mixtures were placed in a 50 mL reactor and kept under mechanical stirring at 100 rpm for 2 hours. The obtained solutions were centrifuged at 3400*g for 10 minutes to obtain air bubble-free solutions and introduced into glass syringes with a Nordson Optimus fluid dispenser, then sterilized for 20 min at 121 °C in an autoclave.

The hydrophilic polymer E.1. used in the following Examples 2-4 is a hydrophilic polymer E.1. comprising a polysaccharide derived from chitosan E.1.1. wherein Rc is DOTAGA, Z is a single bond, x is about 0,06 and y is about 0,17, and further comprising chitosan E.1.1.1. in a ratio E.1.1. : E.1.1.1. of 2:1, said chitosan E.1.1.1. having an acetylation ratio

of less than 10%.

**EXAMPLE 2:** Radiolabeling of the polymer of the system of the disclosure and stability of the pharmaceutical composition E.3.

Different radionuclides were tested to radiolabel the hydrophilic polymer E.1. as described in Example 1:

- Gallium-68 solution; an elution drawn from a Germanium-68 generator by passage of a 0.1 M hydrochloric acid solution.
- Yttrium-90 citrate pharmaceutical solution (YTTRIUM CITRATE (90Y) CIS bio international).
- Fluor-18 solution ; a fluoride solution produced on site by a cyclotron (Curium PET France) in an aqueous solution enriched in $^{18}O$.

Table 1: List of radionuclides used in the examples described

| Radionuclides | Main type of emission | Valence | Physical half-life | Follow-up imaging | Activity used |
|---|---|---|---|---|---|
| $^{68}$**Ga** | $\beta^+$ | $3^+$ | 67 min | TEP | 20 MBq |
| $^{90}$**Y** | $\beta^-$ | $3^+$ | 2.7 j | TEP | 7 MBq |
| $^{18}$**F** | $\beta^+$ | - | 110 min | TEP | 25 MBq |

The hydrophilic polymer E.1 as described in Example 1 and the radionuclide are mixed by coupling 2 syringes *via* a Luer-Lock screw connector. Homogenization of the 2 compounds is achieved at room temperature after about 100 back-and-forth movements of the pistons of the 2 syringes.

The stability of radiolabeling of the pharmaceutical composition E.3. is assessed by measuring the activity present in E.3. and in the supernatant, respectively, over several hours to several days, depending on the half-life of the isotope used.

After the radiolabeling step, 1 mL of radiolabeled composition E.3. is placed in 4 mL of an aqueous medium at pH 7 to 7.4. Two solvents were tested: i) DPBS (Dulbecco's Phosphate Buffered Saline Modified without calcium chloride and magnesium chloride, liquid, sterile-filtered, suitable for cell culture, Sigma-Aldrich) or ii) CSF (Harvard Apparatus Artificial Cerebrospinal Fluid (aCSF)).

After one hour, the volume of solvent not incorporated into composition E.3., i.e. the supernatant, is pipetted off with a p200 pipette and placed in a clean and closed plastic tube (hemolysis tube). The tube is placed in an activimeter (Capintec CRC-25R) to measure the activity present in the supernatant. Similarly, the activity present in composition E.3. is also measured. The supernatant is then brought back into contact with composition E.3.. The measurement is then repeated in the same way several hours later, until the activimeter's measuring limit is reached.

Measurement of the activity present in composition E.3. radiolabelled with Gallium-68 in DPBS medium at the end of the procedure showed a grafting yield of over 95% (17.8 MBq / 18.2 MBq actually used). The labelling yield during the first hour after preparation showed that 90% of the activity is still present in composition E.3. after 60 minutes (Figure 1A).

Radioactivity measurements in composition E.3. radiolabelled with Yttrium-90 in CSF medium and the supernatant, respectively, were carried out at 1, 73, 124 and 168 hours. The results demonstrated the absence of any release of composition E.3. activity into the aqueous medium over time (Figure 1B).

Radioactivity measurements in composition E.3. radiolabeled with Fluor-18 in DPBS and the supernatant, respectively, were carried out at 1, 73, 124 and 168 hours. The results demonstrated the absence of any release of composition E.3. activity into the aqueous medium over time (Figure 1C).

Altogether, these results confirm the possibility of chelating a radioactive trivalent cation in the DOTAGA cages of hydrophilic polymer E.1 and show that the radioactivity contained in compound E.3 is maintained over time.

**EXAMPLE 3:** in vivo analysis.

The same polysaccharide E.1.1. has been used in Example 3 as in Example 2. The inventors have performed positron emission tomography (PET) of Yttrium-90 radiolabeled composition E.3. in a rat surgical excision cavity. The possibility of imaging composition E.3. radiolabeled with Yttrium-90 was verified in vivo in the syngeneic Fischer/F98 rat glioblastoma model. For the procedure, the rat's head was held in place by placing it in a stereotaxic frame. The skin of the skull was incised over approximately 15mm. The aim was then to open the skull by creating a bone flap. After opening, an

excision cavity in the cortex was created by aspiration. Into this cavity, 20 μL of composition E.3. radiolabeled with Yttrium-90 were deposited on the stereotaxic frame using an adapted motorized microinjector. At the end of the operation, after rinsing the surgical area, the bone flap was replaced and held in place with surgical glue. Finally, the skin was sutured with non-absorbable surgical thread. The animal was then placed in a clean cage, on a plate thermoregulated at 37°C, until fully awake. A positron emission tomography (PET) image coupled with computed tomography (CT) of a rat brain 1 hour after the deposition of 1 MBq of the Yttrium-90 radiolabeled composition E.3. in the cerebral cortex was obtained. Results show localized activity in the sensorimotor cortex in the right brain hemisphere. No activity was visible in other part of the body which was acquired (i.e. from brain to the upper part of the kidneys). Results are shown in Figure 2 and Table 2.

Table 2: Activity profile along an axis from the center of the deposited gel and brain parenchyma, measured on a rat PET image acquired one hour after E.3. deposition.

| Distance from center_(mm) | Activity concentration (MBq/mL) |
|---|---|
| 0 | 5.79361 |
| 0.855 | 4.60701 |
| 1.71 | 2.40636 |
| 2.565 | 0.86044 |
| 3.42 | 0.13463 |
| 4.275 | 0 |
| 5.13 | 0 |

The inventors have then performed a dosimetry study. Through successive PET imaging session, it is possible to compute absorbed dose in the resection margins as well as in elimination organs (kidneys, liver...). In addition, by simulation, it is also possible to compare dose spatial distribution of the proposed radiolabeled compound to the previously proposed strategies.

In that way, we used computed tomography (CT) and magnetic resonance imaging (MRI) images from one actual patient available in our laboratory database. CT was registered to MRI then; tumor was segmented on MRI image and the mask was carried over onto the registered CT. Tissue density into the mask was set to cerebrospinal fluid to mimic tumor resection. This pseudo "post-surgery" CT was then used as a geometry in Gate 9.3 (running Geant4 11.03) software with a voxel resolution of 1x1x1 mm3. A dose actor was set to record the absorbed dose in the brain with the same spatial resolution as the defined geometry. We used the emstandard_opt4 physics list model and cuts were set at xx μm for electrons (primary and secondary). Other cuts were left at their default values.

Three different sources were defined to compare Gliasite®, GammaTile® and proposed composition E.3. respectively.

1. Control (no radioactive source).
2. Gliasite was defined as a sphere filled with 125I aqueous solution.
3. GammaTile was defined as sets of 4 131Cs seeds contained into titanium cases. Complete geometry of these seeds has been extracted from GammaTile device product information sheet, ref GT-001 (GT Medical, Tempe, AZ).
4. Composition E.3. source was defined as a chitosan gel filling the resection cavity with a homogeneous 90Y distribution.

For each configuration, simulations were performed with $1e^8$ primary events. Then dose maps were expressed in μGy.MBq-1.s-1. The results (shown in Figure 3) show a very high and localized radioactivity with the composition E.3. while the radioactivity is lower with Gliasite, with some release of the radioactivity to the nerby tissues. The radioactivity observed with GammaTile is much lower and also diffuse outside the site of injection of the source. Gliasite dose distribution is defined within a sphere, due to balloon inflation. GammaTile distribution revealed a heterogeneous dose distribution around the four seeds placed into the collagen patch. Composition E.3. dose distribution respects more the tumor shape (visible on CT image) and deposited dose rate per MBq is higher with $^{90}Y$ than with $^{131}Cs$ which is used in GammaTile.

**EXAMPLE 4:** Gelation of composition E.3. of chitosan@DOTAGA

The exemplified composition comprises a polysaccharide E.1.1. having DOTAGA as Rc group and a chitosan E.1.1.1. at a ration E.1.1./E.1.1.1. of 2/3. The composition is injected in PBS (phosphate buffered saline) to mimic

physiological conditions and analyze the gelation of the composition.

The viscoelastic properties reflecting the kinetics of hydrogel formation of the composition E.3. were first assessed *in vitro* for this composition after immersion in a phosphate-saline buffer mimicking the physiological environment (PBS medium - pH = 7.4, osmolarity = 300 mOsm/L). Approximately 2 mL of the composition was placed into a round mold with an inner diameter of 25 mm and a height of 3 mm. The mold containing the composition was then immersed in 50 mL of 10 mmol/L PBS to initiate the gelation process of the composition. The elastic modulus (G') and viscous modulus (G") of the formed hydrogel were recorded on an ARES rheometer (TA instruments) using a 25 mm plate geometry. Prior to measurement, the zero fixture was achieved by placing an empty mold with double-sided tape on the lower part of the geometry. A preliminary study was carried out on hydrogels with a dynamic strain sweep test by varying the deformation from 0.1% to 100% at a fixed frequency of 10 rad/s at 25°C to ensure that the measurements are carried out in the linear regime of the material studied. The rheological properties of the formed hydrogel were determined measured on the placed mold containing the hydrogel with a dynamic frequency sweep test from 0.05 rad/s to 100 rad/s at a constant strain of 1% at 25°C. For the kinetics study, measurement at different time points were achieved on different gel to ensure that the measurement would not affect rheological properties of the material.

As shown in Figure 4, the composition gels after injection in the medium and spontaneously becomes a hydrogel with a targeted elastic modulus G' and damping factor tan($\delta$) under physiological conditions.

**EXAMPLE 5:** Hydrophilic polymer E.1. comprising a polymer based on hyaluronic acid

This example describes the implementation of hydrophilic polymer E.1. comprising a polymer based on hyaluronic acid grafted with DOTAGA as chelating agent.

Sodium hyaluronate (Streptococcus equi ; 500-750kDa) was purchased from Matexcel. Other reagents such as 1-ethyl-3-(3-dimethylaminopropyl)carbodiimide (EDC), hydroxybenzotriazole (HOBt), N-methylmorpholine (NMM), 2-chloro-4,6-dimethoxy-1,3,5-triazine (CMDT), 4-(4,6-dimethoxy-1,3,5-triazin-2-yl)-4-methylmorpholinium chloride (DMTMM), etc., were obtained of analytical grade from Sigma-Aldrich and used as received without further purification. The water used is milli-Q ultrapure water (18.2 M$\Omega$).

**Grafting of the ethylenediamine linker**

*Carbodiimide synthesis*

The synthesis was carried out by solubilizing 1.07g (2.65mmol) of hyaluronic acid in a flask containing 100mL of water. 2.64mL (39.6mmol) of ethylene diamine were added and the pH adjusted to 7 by controlled addition of 1M hydrochloric acid. 355mg (2.63mmol) of HOBt was introduced into the reaction medium, followed by 504mg (2.63mmol) of EDC. EDC was added after HOBt to avoid the formation of the stable reaction intermediate N-acyl urea which would prevents the linker from forming an amide bond with hyaluronic acid. The pH was then adjusted and kept at 5.5 for 1h by the controlled addition of a 1M HCl solution, allowing activation of the reagents by preserving a small part of the acid in its protonated form on which the EDC could react. The pH was then adjusted to 7.5 and the reaction was left stirring for 5 days. The product was then purified by tangential filtration using the Sartoflow® Advanced device with a Sartocon® Slice PESU cassette (polyethersulfone membranes; cut-off: 300 kDa; filtration area: 0.1 $m^2$) against 20 L of a $10^{-3}$ $mol.L^{-1}$ sodium hydroxide solution, followed by 40 L of milli-Q water. The use of a basic solution with a pH of around 11 neutralizes the charge of ungrafted ethylenediamine (pKa=9.98), which could interact with hyaluronic acid carboxylates and not be eliminated during tangential filtration. Finally, the product was freeze-dried.

Grafting of the ethylenediamine linker onto the hyaluronic acid backbone was quantified by $^1H$ NMR analysis. NMR analyses were performed by solubilizing 7.5mg of the synthesis product in 750 $\mu$L of $D_2O$ solvent containing a 0.75% (w/w) TSPd4 internal standard under stirring for 24 hours. The solution was then introduced into a 4cm high NMR tube, washed with acetone and oven-dried for at least 24 hours. $^1H$ spectra were performed according to the following parameters: T: 353 K, number of scans: 256, pulse program: zg30. Analyses were performed on a Bruker Ascend 400 MHz NMR spectrometer. All spectra were analyzed with Topspin software (V4.1.3), including phase and baseline correction, automatic peak selection and manual peak integration. Chemical shifts were corrected using the standard peak (0 ppm).

The $^1H$ NMR spectrum of native hyaluronic acid shows a singlet peak resolved at around 2.0 ppm corresponding to the three methyl protons bore by the acetyl group of the N-acetyl glucosamine unit. As reactions with EDC/HOBt or DMTMM do not induce deacetylation, this peak will be taken as a reference peak and calibrated to determine the grafting yield of the linker onto hyaluronic acid. It is also noted the presence of a doublet of doublets induced by the presence of the anomeric proton on the hyaluronic acid repeat units. For room-temperature analyses, this signal is generally covered by the HDO peak. Finally, the cluster of peaks between 3.3 ppm and 4.1 ppm corresponds to the other protons present in the rings of the repeat units.

The $^1H$ NMR spectrum of the synthesis product exhibits the same peaks as native hyaluronic acid, except for the singlet at 2.9ppm and the triplet at 3.2 ppm. The singlet peak at 2.9ppm correspond to the ungrafted ethylenediamine electrostatically bind to carboxylate of the hyaluronic acid backbone. The 3.2 ppm triplet peak results from ethylene diamine grafting, where the 2 protons of the methylene group are no longer equivalent due to the formation of the amide bond and consequent loss of symmetry in the molecule. Therefore, by integrating this triplet and applying the following formula, we can easily determine the grafting yield of the linker: $x = \frac{\delta_{3.2ppm}/2}{\delta_{2.0ppm}/3} = 14.6\%$ .

*Triazine synthesis*

The synthesis was performed by solubilizing 2.02g (5.03mmol) of hyaluronic acid in 100mL of water. 357μL (5.34mmol) of ethylene diamine was first added to the solution, adjusting the pH to 6.5 by controlled addition of 1M HCl solution. Next, 1.48g (5.35mmol) of DMTMM was introduced into the reaction medium and left stirring for 48h. The product was then purified by tangential filtration using the Sartoflow® Advanced device with two Sartocon® Slice PESU cassettes (polyethersulfone membranes; cut-off: 100 kDa; filtration area: 0.1 $m^2$) against 10 L of a $10^{-3}$ $mol.L^{-1}$ sodium hydroxide solution, followed by 20 L of milli-Q water. Finally, the product was freeze-dried. A grafting degree of 37.1% was determined by $^1H$ NMR analysis.

**Grafting of DOTAGA**

500mg of hyaluronic acid modified with ethylene diamine was added to 50mL of distilled water and left under stirring until the polymer was completely solubilized. 2g of DOTAGA anhydride was then added to the reaction medium and the mixture was left under stirring for 24h. The product was then purified by tangential filtration using the Sartoflow® Advanced device with two Sartocon® Slice PESU cassettes (polyethersulfone membranes; cut-off: 100 kDa; filtration area: 0.1 $m^2$) against 40 L of milli-Q water. Finally, the product was freeze-dried.

DOTAGA grafting degree onto the ethylenediamine free amine was estimated by NMR $^1H$ with the difference in peak integration at 3.2ppm between the product before and after DOTAGA grafting, where DOTAGA after grafting contributes to this signal with two protons carried by a methylene group with a chemical shift also around 3.2ppm. For a 10.2% ethylenediamine graft via carbodiimide using a protocol similar to that described in part 1) a), a 4.7% DOTAGA graft was obtained.

**HA@DOTAGA crosslinking with BDDE**

500 mg of HA@DOTAGA (polymer E.1. comprising a polysaccharide derived from hyaluronic acid grafted with DOTAGA as described above) were solubilized in 4.95mL of 0.25M NaOH and stirred until the polymer was completely dissolved. Next, 50μL of BDDE (1,4-Butanediol diglycidyl ether) was added to the reaction medium with vigorous stirring, and stirring was maintained for 30 minutes to homogenize the reaction medium. The medium was then placed in an oven at 50°C for 3h. The hydrogel was then removed from the oven and placed in a suitable pot with 20mL of distilled water to obtain a total hyaluronic acid concentration of 20mg/g. The gel was fragmented using a T 25 digital ULTRA-TURRAX® high-performance disperser for 10 minutes at 10,000 rpm and the medium was then neutralized by adding a 1M HCl solution to reach a pH of 7. The gel was then dialyzed (cut-off 300kDa) for 48h against a 10mM PBS solution where the medium was regularly renewed to ensure that residual BDDE was eliminated during this step.

Such hydrophilic polymer E.1. comprising a hyaluronic acid polymer bearing DOTAGA as chelating agent can be radiolabeled in a similar way as described in Example 2 above and used as a pharmaceutical composition of the invention.

**Claims**

1. A system for preparing a pharmaceutical radioactive hydrogel comprising:

   **E.1.** at least one hydrophilic polymer E.1. bearing at least one chelating group, having ability to become or remain a hydrogel under physiological conditions,
   **E.2.** at least one therapeutical radionuclide E.2. chelatable with said chelating group, and preferably under the form of a liquid, and more preferably under the form of an aqueous solution of radioactive cations.

2. The system according to claim 1 wherein said hydrophilic polymer E.1. is a polysaccharide E.1.1. derived from chitosan of formula (I):

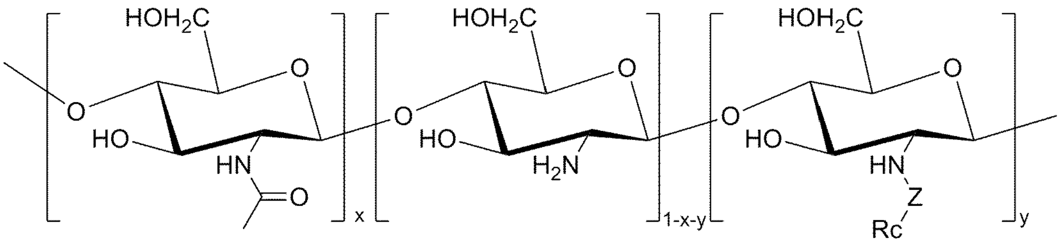

(I)

wherein:

- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- x is comprised between 0 and 0.1, preferably between 0.005 and 0.09, more preferably between 0.02 and 0.08,
- y is comprised between 0.01 and 0.4, particularly between 0.05 and 0.35, preferably between 0.08 and 0.3, more preferably 0.1 and 0.25, even more preferably between 0.1 and 0.20,
- y/x is comprised between 0.2 and 50, particularly between 0.5 and 45, preferably between 0.8 and 40, more preferably between 1 and 35, even more preferably between 1.5 and 30.
- x + y is comprised between 0.1 and 0.5, particularly between 0.2 and 0.45, preferably between 0.2 and 0.4.

3. The system according to claim 2 wherein said hydrophilic polymer further comprises at least one chitosan E.1.1.1, particularly in a ratio E.1.1. : E.1.1.1. between 5:1 and 1:5, preferably between 3:1 and 1:3, more preferably between 3:1 and 1:1, typically of about 2:1..

4. The system according to claim 3 wherein said chitosan E.1.1.1. as a proportion of acetylation of less than 10%.

5. The system according to claim 1 wherein said hydrophilic polymer E.1. is a polysaccharide E.1.2. derived from hyaluronic acid of formula (II):

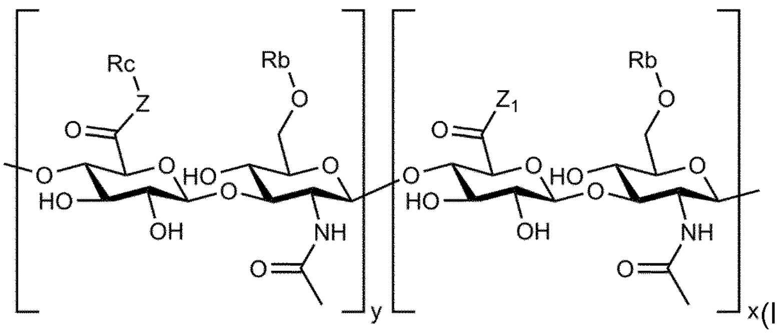

(II)

wherein:

- Rb is a crosslinking group,
- Rc is a chelating group,
- Z is a linker which may be a single bond or a hydrocarbon-based chain comprising between 1 and 12 carbon atoms, said chain being linear or branched, saturated or unsaturated and optionally comprising one or more heteroatoms, preferably selected from nitrogen, oxygen, sulfur, atoms of the halogen family, and combinations thereof,
- Z1 a group comprising an hydroxyl function
- x is comprised between 0.6 and 0.95, preferably between 0.7 and 0.85, more preferably between 0.75 and 0.8,
- y is comprised between 0.05 and 0.4, preferably between 0.15 and 0.3, more preferably 0.15 and 0.2.

6. The system according to any one of claims 1 to 5, wherein the chelating group is selected from the group DOTA, NOTA, NODAGA, DOTAGA, DOTAM, NOTAM, DOTP, NOTP, TETA, TETAM, DTPA, EDTA and DFO, particularly from the

group consisting of DOTAGA, DFO, DOTAM and DTPA, preferably DOTAGA.

**7.** The system according to any one of claims 1 to 6, wherein the therapeutical radionuclide E.2. is selected from Bismuth-213, Cesium-131, Holmium-166, Iridium-192, Lutetium-177, Mercury-197, Rhenium-186, Rhenium-188, Yttrium-90, Terbium-155, Terbium-149, Terbium-161, Astatine-211, Actinium-225, Lead-212, Radium-223, Copper-64, Copper-67, particularly Yttrium-90, Lutetium-177 or Actinium-225. Preferably, the therapeutical radionuclide E.2. is Yttrium-90.

**8.** The system according to any one of claims 1 to 4 and 6 to 7, comprising:

**E.1.** a hydrophilic polymer E.1. under the form of an aqueous solution comprising:

E.1.1. at least one statistical polysaccharide E.1.1. of formula (I) having a weight-average molecular weight of between 100 kDa and 1000 kDa wherein

- Rc is DOTAGA,
- Z is a single bound,
- x is between 0.02 and 0.08, for example 0.06,
- y is between 0.10 and 0.20, for example 0,17,

E.1.2. at least one chitosan E.1.1.1. with less than 10% acetylation present in a ratio E.1.1. : E.1.1.1. of 2:1; and

**E.2.** an aqueous solution comprising Yttrium-90 radionuclide E.2. chelatable with DOTAGA of E.1.1..

**9.** The system according to any one of claims 1 and 5 to 7, comprising:

**E.1.** a hydrophilic polymer under the form of an aqueous solution comprising at least one polysaccharide E.1.2. of formula (II) having a weight-average molecular weight of between 5000 kDa and 7000 kDa wherein

- Rb is BDDE,
- Rc is DOTAGA,
- Z is ethylendiamine,
- Z1 is OH,
- x is between 0.75 and 0.85, for example 0,77,
- y is between 0.15 and 0.25, for example 0.23, and

**E.2.** an aqueous solution comprising Yttrium-90 radionuclide E.2. chelatable with DOTAGA of E.1.2..

**10.** A pharmaceutical composition E.3. as part of the system for preparing a pharmaceutical radioactive hydrogel according to claims 1 to 9 comprising said hydrophilic polymer E.1. including said therapeutical radionuclide E.2. chelated with the chelating group Rc contained in E.1..

**11.** The pharmaceutical composition E.3. according to claim 10, said pharmaceutical composition being injectable.

**12.** A pharmaceutical radioactive hydrogel susceptible to be obtained from the system according to any one of claims 1 to 9 or the pharmaceutical composition E.3. according to any one of claims 10 or 11 under physiological conditions.

**13.** The pharmaceutical radioactive hydrogel according to claim 12 having a damping factor tan($\delta$) between 0,01 and 1 and/or an elastic modulus G' between 20 Pa and 3000 Pa under physiological conditions.

**14.** The pharmaceutical composition E.3. according to any one of claims 10 to 11 for use as a drug.

**15.** The pharmaceutical composition E.3. according to any one of claims 10 to 11 and 14 for use in the treatment of solid cancer, preferably by brachytherapy.

**16.** The pharmaceutical composition E.3. according to any one of claims 10 to 11 and 14 to 15 wherein the solid cancer is selected from liver cancer, prostate cancer, breast cancer, skin cancer/melanoma, glioblastoma, preferably glioblastoma.

**17.** A method for preparing a pharmaceutical composition E.3. according to any one of claims 10 to 11 comprising the following steps:

(i) implementing the hydrophilic polymer E.1. of the system according to any one of claims 1 to 8;
(ii) implementing the therapeutical radionuclide E.2. of the system according to any one of claims 1 to 8;
(iii) Mixing said hydrophilic polymer E.1. with said therapeutical radionuclide E.2., allowing the chelation of said therapeutical radionuclide E.2. with the chelating group(s) present in the hydrophilic polymer E.1.;

wherein such solution obtained at step (iv) allows formation of a hydrogel, particularly under physiological conditions.

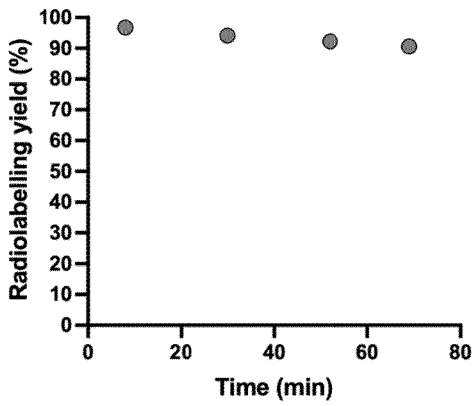
Radiolabelling yield (%)
100
90
80
70
60
50
40
30
20
10
0
0
20
40
60
80
Time (min)

A.

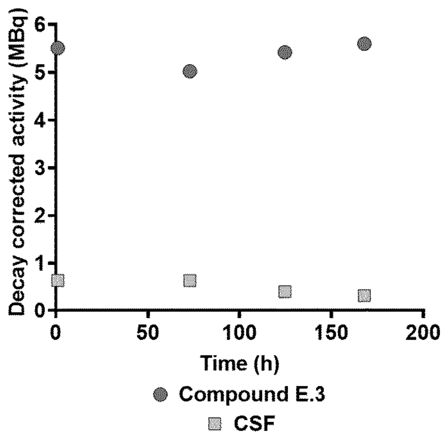
Decay corrected activity (MBq)
6
5
4
3
2
1
0
0
50
100
150
200
Time (h)
Compound E.3
CSF

B.

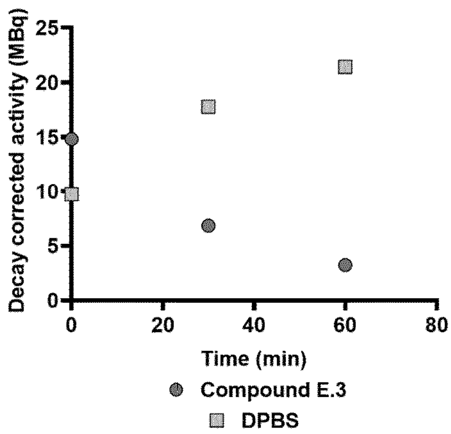
Decay corrected activity (MBq)
25
20
15
10
5
0
0
20
40
60
80
Time (min)
Compound E.3
DPBS

C.

Figure 1

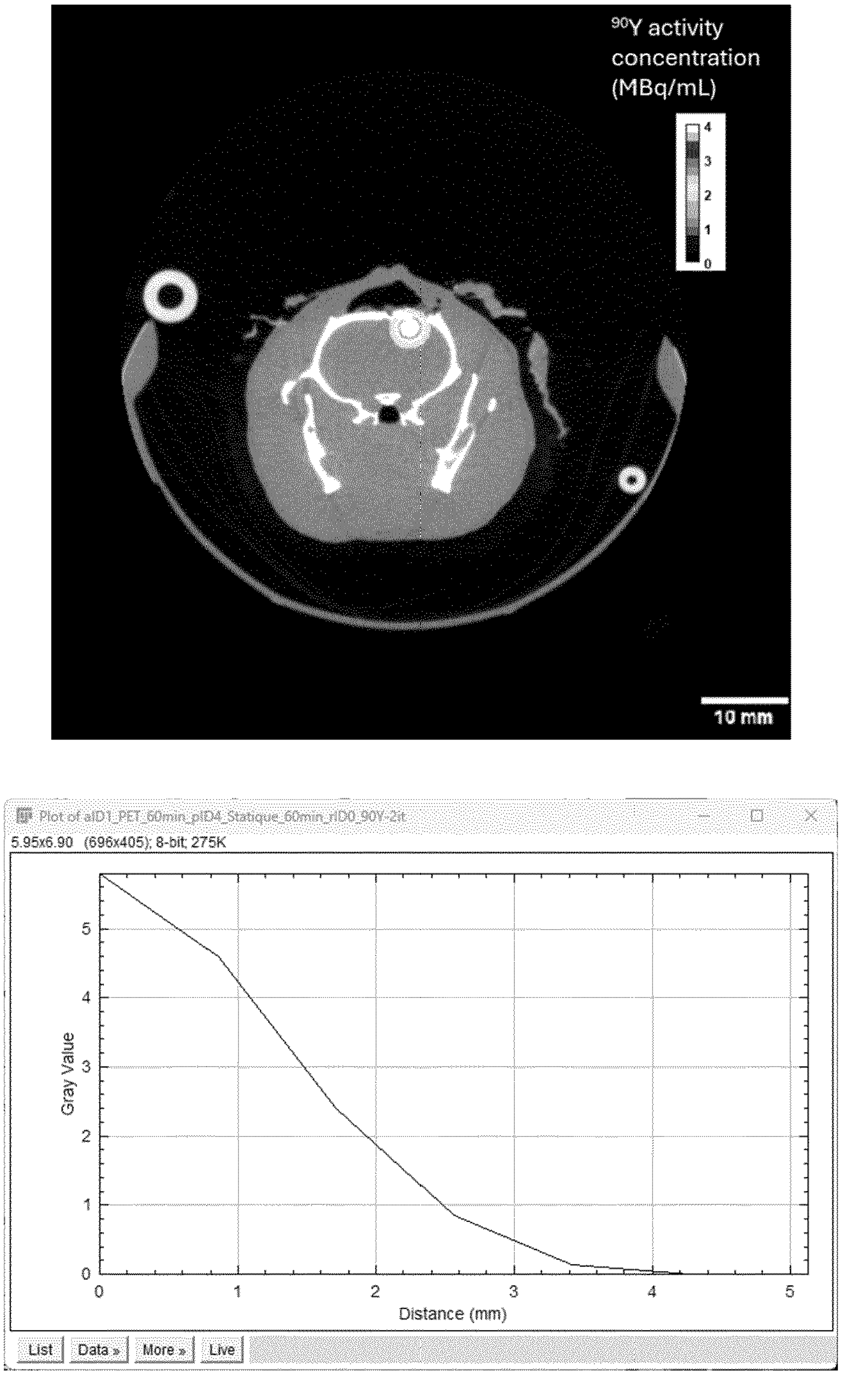

$^{90}Y$ activity concentration (MBq/mL)
4
3
2
1
0
10 mm
Plot of aID1_PET_60min_pID4_Statique_60min_rID0_90Y-2it
5.95x6.90 (696x405); 8-bit; 275K
Gray Value
Distance (mm)
0
1
2
3
4
5
List
Data »
More »
Live


Figure 2

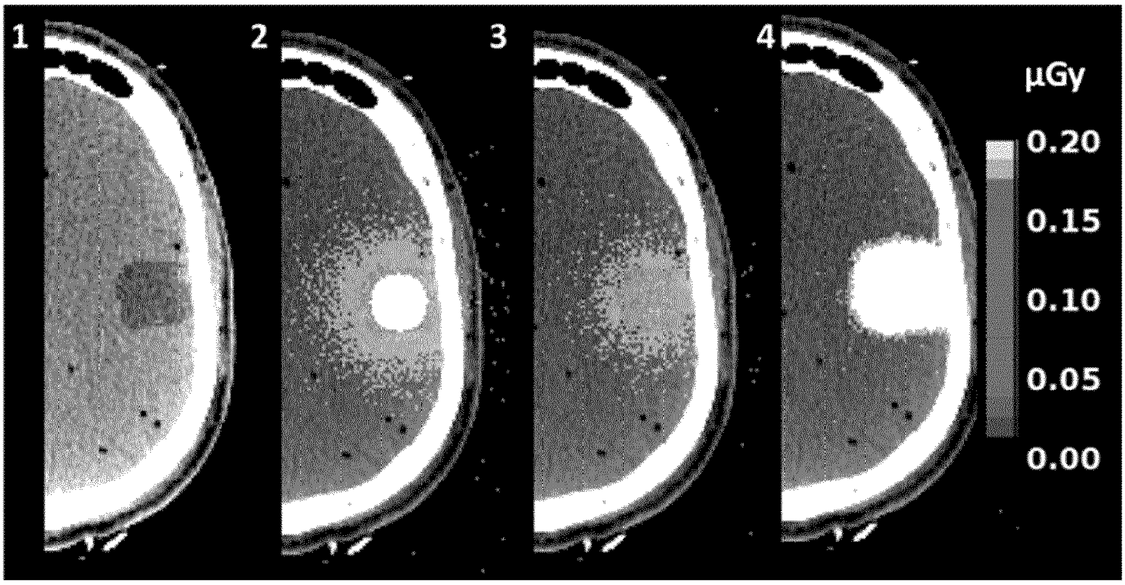


Figure 3

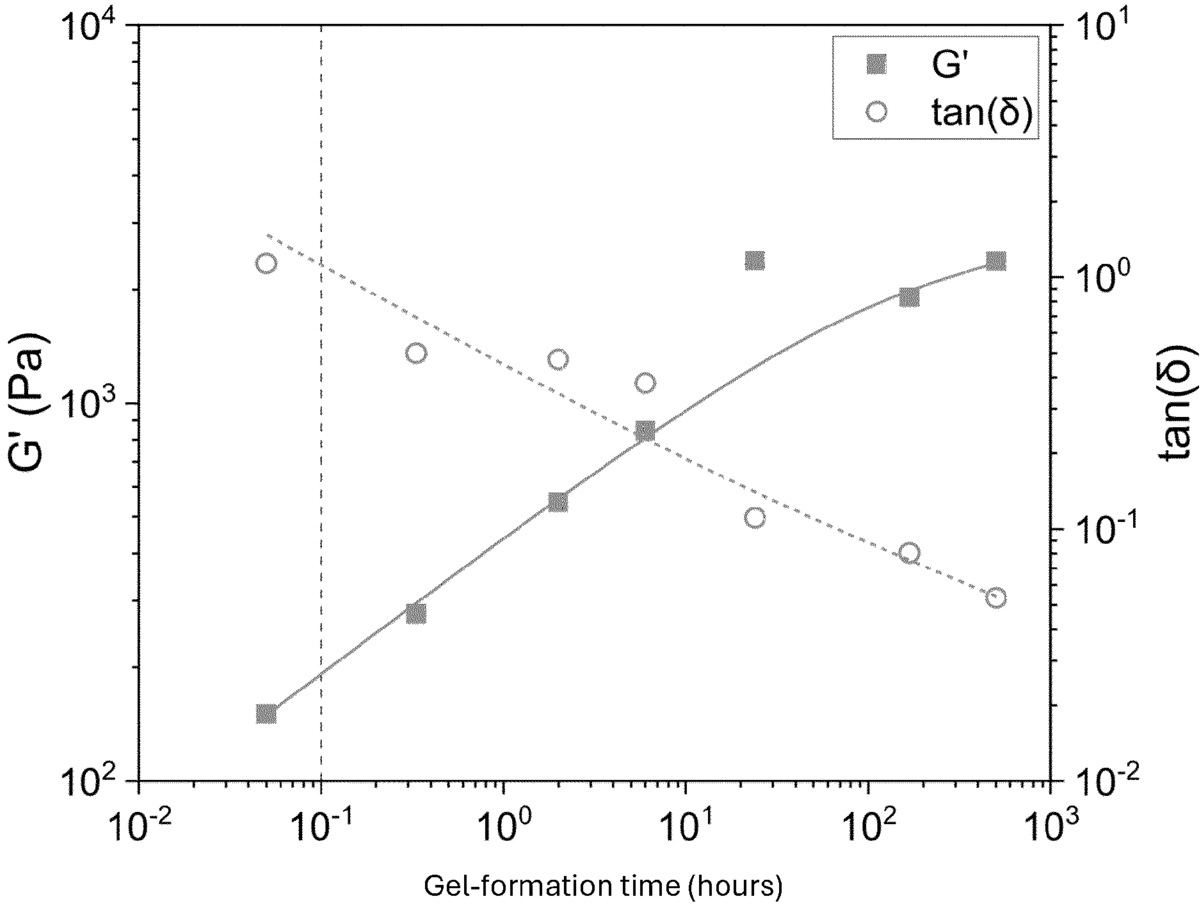


Figure 4

Europäisches Patentamt
European Patent Office
Office européen des brevets

# EUROPEAN SEARCH REPORT

Application Number
EP 24 30 7284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/023677 A1 (MEXBRAIN [FR]; CENTRE NAT RECH SCIENT [FR] ET AL.) 3 February 2022 (2022-02-03) | 1-4,6,7, 10-17 | INV. A61K51/06 A61K51/12 A61P35/00 A61K103/32 |
| A | * paragraph [0012] * * paragraph [0015] * * paragraph [0085] * * paragraph [0097] - paragraph [0098] * * examples 1-4, 8 * ----- | 5,8,9 | |
| X | WO 2021/153823 A1 (KAI BIOTECH CO LTD [KR]) 5 August 2021 (2021-08-05) | 1,6,7, 10-17 | |
| A | * paragraph [0001] * * paragraph [0056] * * examples 1-3 * ----- | 2-5,8,9 | |
| X | EP 4 389 804 A1 (NAT UNIV PUSAN IND UNIV COOP FOUND [KR]) 26 June 2024 (2024-06-26) | 1,5-7, 10-17 | |
| A | * example 2 * * paragraph [0004] - paragraph [0005] * * paragraph [0018] - paragraph [0025] * ----- | 2-4,8,9 | TECHNICAL FIELDS SEARCHED (IPC) |
| X | CN 107 488 240 A (INST NUCLEAR PHYSICS & CHEMISTRY CHINA ACAD ENG PHYSICS) 19 December 2017 (2017-12-19) | 1,7, 10-15,17 | A61K A61P |
| A | * examples 1-9 * * paragraph [0043] * * paragraph [0006] - paragraph [0018] * ----- | 2-6,8,9, 16 | |

The present search report has been drawn up for all claims

1

| Place of search | Date of completion of the search | Examiner |
|---|---|---|
| The Hague | 28 May 2025 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 30 7284

This annex lists the patent family members relating to the patent documents cited in the above-mentioned European search report. The members are as contained in the European Patent Office EDP file on
The European Patent Office is in no way liable for these particulars which are merely given for the purpose of information.

28-05-2025

| Patent document cited in search report | Publication date | Patent family member(s) | Publication date |
|---|---|---|---|
| WO 2022023677 A1 | 03-02-2022 | CN 116157465 A | 23-05-2023 |
| | | EP 4188966 A1 | 07-06-2023 |
| | | FR 3112943 A1 | 04-02-2022 |
| | | JP 2023535504 A | 17-08-2023 |
| | | US 2023263912 A1 | 24-08-2023 |
| | | WO 2022023677 A1 | 03-02-2022 |
| WO 2021153823 A1 | 05-08-2021 | CN 115038467 A | 09-09-2022 |
| | | KR 20220123260 A | 06-09-2022 |
| | | WO 2021153823 A1 | 05-08-2021 |
| EP 4389804 A1 | 26-06-2024 | EP 4389804 A1 | 26-06-2024 |
| | | JP 2024534101 A | 18-09-2024 |
| | | US 2024352199 A1 | 24-10-2024 |
| | | WO 2023022554 A1 | 23-02-2023 |
| CN 107488240 A | 19-12-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5762903 A
- WO 2023148351 A1


### Non-patent literature cited in the description


- **RINGEL F et al.** *Neuro Oncol*, 2016, vol. 18, 96-104
- **A. MONTEMBAULT** ; **C. VITON** ; **A. DOMARD**. Physico-chemical studies of the gelation of chitosan in a hydroalcoholic medium. *Biomaterials*, 2005, vol. 26 (8), 933-943
- **A. HIRAI** ; **H ODANI** ; **A. NAKAJIMA**. *Polymer Bulletin*, 1991, vol. 26 (1), 87-94
- X-ray Diffraction Methods. **ALEXANDER, L. E.** Polymer Science. Wiley-Interscience, 1969, 137
- **WATT RP et al.** *Inet J Pharm*, 10 January 2019, vol. 554, 376-386
- **El KISSI et al.** *Rheology*, 2006, vol. 10, 13-39
- **NATUZZI et al.** *Nature Scientific Reports*, 2021, vol. 11, 19948
- **SCHATZ et al.** *Biomacromolecules*, 2003, vol. 4 (3), 641-8
- **HIRAI et al.** *Polymer Bulletin*, 1991, vol. 26, 87-94